# EUROPEAN PATENT SPECIFICATION

(11) **EP 4 010 066 B1**
(45) Date of publication and mention of the grant of the patent: **15.05.2024**
(21) Application number: 20849024.3
(22) Date of filing: 05.08.2020
(51) Int. Cl.: A61N 1/36, A61N 1/05, A61N 1/04

(54) **SYSTEMS AND METHODS FOR MONITORING NEURAL ACTIVITY**
SYSTEME UND VERFAHREN ZUR ÜBERWACHUNG DER NERVENAKTIVITÄT
SYSTÈMES ET PROCÉDÉS DE SURVEILLANCE DE L'ACTIVITÉ NEURONALE

(30) Priority: 06.08.2019 AU 2019902808
(43) Date of publication of application: 15.06.2022
(73) Proprietor: Deep Brain Stimulation Technologies Pty Ltd, East Melbourne, VIC 3002 (AU)
(72) Inventor: SINCLAIR, Nicholas, East Melbourne, Victoria 3002 (AU); MCDERMOTT, Hugh, East Melbourne, Victoria 3002 (AU); THEVATHASAN, Arthur Wesley, East Melbourne, Victoria 3002 (AU); BULLUSS, Kristian, East Melbourne, Victoria 3002 (AU); FALLON, James, East Melbourne, Victoria 3002 (AU)
(74) Representative: Haseltine Lake Kempner LLP
(86) International application number: PCT/AU2020/050806
(87) International publication number: WO 2021/022332

(56) References cited:
- WO-A1-2018/027259
- WO-A1-2018/213872
- WO-A1-2018/213872
- WO-A1-2019/210371
- US-A1- 2018 333 582
- SINCLAIR NICHOLAS C ET AL: "On the neural basis of deep brain stimulation evoked resonant activity", BIOMEDICAL PHYSICS & ENGINEERING EXPRESS, vol. 5, no. 5, 7 August 2019 (2019-08-07), pages 057001-1, XP055791417, DOI: 10.1088/2057-1976/ab366e Retrieved from the Internet: URL:https://iopscience.iop.org/article/10. 1088/2057-1976/ab366e>
- GMEL, G. et al.: "A new biomarker for subthalamic deep brain stimulation for patients with advanced Parkinson's disease-a pilot study", Journal of Neural Engineering, vol. 12, no. 6, 2015, pages 1-11, XP020293217, DOI: 10.1088/1741-2560/12/6/066013
- Sinclair Nicholas C, Fallon James B, Bulluss Kristian J, Thevathasan Wesley, Mcdermott Hugh J: "On the neural basis of deep brain stimulation evoked resonant activity", Biomedical Physics & Engineering Express, vol. 5, no. 5, 7 August 2019 (2019-08-07), pages 1-8, XP055791417,
- LI, S. et al.: "Resonant Antidromic Cortical Circuit Activation as a Consequence of High-Frequency Subthalamic Deep-Brain Stimulation", Journal of Neurophysiology, vol. 98, 2007, pages 3525-3537, XP055343568, DOI: 10.1152/jn.00808.2007

## Description

### Technical Field

The present disclosure relates to deep brain stimulation (DBS) and, in particular, methods and systems of monitoring neural activity responsive to DBS.

### Background

Deep brain stimulation (DBS) is an established therapy for movement disorders as well as other neurological disorders, including epilepsy, obsessive compulsive disorder, and depression. DBS is typically administered to patients whose symptoms cannot be adequately controlled by medication alone. DBS involves surgically implanting electrodes in or near to specific neural structures of the brain, typically in the subthalamic nucleus (STN), the globus pallidus interna (GPi), and/or the thalamus. Electrodes are connected to a neurostimulator usually implanted within the body and configured to deliver electrical pulses into target areas. It is believed that this electrical stimulation disrupts abnormal brain activity causally linked to a patient's symptoms. Stimulation parameters can be adjusted using a controller external to the body, remotely connected to the neurostimulator.

Whilst established DBS technology has proven to be effective in alleviating movement disorder symptoms, there are several limitations to state of the art devices. In particular, established techniques for intraoperative testing of DBS electrodes to ensure correct positioning in the brain, such as x-ray imaging, microelectrode recordings, and clinical assessment can be inaccurate. Consequently, electrodes are often implanted in suboptimal locations, resulting in diminished therapeutic outcomes and unwanted side-effects. After implantation, DBS devices require manual adjustment by a clinician. This typically involves the clinician adjusting parameters of the stimulus based on a largely subjective assessment of immediate or short-term improvement of the patient's symptoms. Since therapeutic effects can be slow to emerge and because the DBS parameter space is large, the task of finding a preferred set of parameters is time- and cost-inefficient, and can lead to suboptimal therapeutic outcomes. In addition, the constant, non-varying application of electrical stimulation using conventional DBS can also lead to suboptimal therapeutic outcomes, including unwanted side effects, as well as reduced battery life of DBS stimulators.

WO 2018/213872 A1 describes a method of monitoring neural activity comprising detecting high frequency oscillations (HFOs) between about 200 Hz and about 500 Hz and determining waveform characteristics of the detected HFOs.

### Summary

According to a first aspect of the disclosure, there is provided a non-therapeutic method, comprising: a) applying a plurality of electrical stimuli having differing amplitudes via a first electrode at a first position in a brain of a patient, a total energy provided to the patient by the plurality of electrical stimuli being below a therapeutic threshold; for each of the plurality of electrical stimuli; b) detecting a resonant response from a target neural structure in the brain, each resonant response evoked by a respective one of the electrical stimuli; c) determining a change in one or more common waveform characteristics between two or more of the detected resonant responses; d) determining an effectiveness of in delivering therapeutic stimulation at the first position based on the change.

The one or more common waveform characteristics may comprises one or more of the following: an amplitude of the resonant response; a resurgence of the resonant response; a frequency of the resonant response; a temporal envelope of the resonant response; a fine structure of the resonant response; a rate of decay of the resonant response; a delay between the onset of the stimulus and the onset of a temporal feature of the resonant response amplitude.

Determining the change in the one or more common waveform characteristics between the two or more of the detected resonant responses may comprise determining a rate of change in the one or more of the common waveform characteristics between the two or more of the detected resonant responses

The method may further comprise selecting an amplitude of the differing amplitudes for therapeutic stimulation of the target neural structure based on the change.

The method may further comprise selecting the first position for therapeutic stimulation of the target neural structure based on the change.

The method may further comprise repeating a-d at a second position in the brain different from the first position; comparing the changes in the one or more common waveform characteristics between the first and second positions. The method may further comprise selecting one of the first position and the second position for therapeutic stimulation of target neural structure based on the comparison. The method may further comprise applying a therapeutic stimulus to the target neural structure at the selected position.

The method may further comprise moving the first electrode between the first position and the second position in the brain; and determining an optimum electrode location for therapeutic stimulation of target neural structure based on the comparison of the changes in the one or more common waveform characteristics between the first and second positions.

The plurality of electrical stimuli applied at the second position may be applied by a second electrode of a plurality of electrodes.

In some embodiments, the plurality of electrical stimuli applied at the first location are applied at the first location by two or more electrodes. In some embodiments, at least one of the two or more electrodes are not located at the first position. In some embodiments, the resonant response is detected at two or more electrodes.

The method may further comprise removing the plurality electrodes from the first position in the brain; and implanting a permanent one or more electrodes at the first position in the brain.

For any or all of the above steps, the patient may under general anaesthetic. In some embodiments, the patient may be under general anaesthetic for at least steps a to d described above.

According to another aspect of the disclosure, there is provided a neurostimulation system, comprising: at least one electrode adapted for implantation in or near a target neural structure in the brain; a signal generator selectively coupled to one or more of the at least one electrode and configured to apply a plurality of electrical stimuli having differing amplitudes via a first electrode of the at least one electrode at a first position in a brain of a patient; a measurement device selectively coupled to one or more of the at least one electrode and configured to detect a resonant response from the target neural structure evoked by each of the plurality of electrical stimuli; and a processing unit coupled to the measurement device and configured to: determine a change in one or more common waveform characteristics between two or more of the detected resonant responses; and determine an effectiveness of delivering therapeutic stimulation at the first position based on the change.

Determining the change in the one or more common waveform characteristics between the two or more of the detected resonant responses may comprise determining a rate of change in the one or more of the common waveform characteristics between the two or more of the detected resonant responses.

The one or more common waveform characteristics may comprise one or more of the following: an amplitude of the resonant response; a resurgence of the resonant response; a frequency of the resonant response; a temporal envelope of the resonant response; a fine structure of the resonant response; a rate of decay of the resonant response; a delay between the onset of the stimulus and the onset of a temporal feature of the resonant response amplitude.

The processing unit may be further configured to select an amplitude of the differing amplitudes for therapeutic stimulation of the target neural structure based on the change. The processing unit may be further configured to select the first position for therapeutic stimulation of the target neural structure based on the change.

The at least one electrode may comprise the first electrode at the first position and a second electrode at a second position different from the first position. The signal generator may be configured to apply the plurality of electrical stimuli to each of the first and second electrodes. The measurement device may be configured to detect the resonant response from the target neural structure evoked by the plurality of electrical stimuli applied to each of the first and second electrodes. The processing unit may be configured to: determine a change in one or more common waveform characteristics between two or more of the detected resonant responses evoked by each of the plurality of electrical stimuli applied to the second electrode in the second position; compare the changes in the one or more common waveform characteristics of the two or more of the detected resonant responses at the first electrode to a change in one or more common waveform characteristics between two or more of the detected resonant responses at the second electrode; and select one of the first electrode and the second electrode for therapeutic stimulation of target neural structure based on the comparison.

The signal generator may be further configured to apply a therapeutic stimulus to the target neural structure via the selected electrode.

The plurality of electrical stimuli are applied to the first electrode of the at least one electrode. In response to moving of the first electrode to a second position in the brain different from the first position, the signal generator may be configured to apply the plurality of electrical stimuli to the first electrode in the second position. The measurement device may be configured to detect the resonant response from the target neural structure evoked by each of the plurality of electrical stimuli applied to the first electrodes in the second position. The processing unit may be configured to: determine a change in one or more common waveform characteristics between two or more of the detected resonant responses evoked by each of the plurality of electrical stimuli applied to the first electrode in the second position; compare the change in the one or more common waveform characteristics between the two or more of the detected resonant responses detected at the first position to a change in the one or more common waveform characteristics between the two or more of the detected resonant responses detected at the second electrode; and determine an optimum electrode position for therapeutic stimulation of target neural structure based on the comparison.

In some embodiments, the plurality of electrical stimuli applied at the first location are applied at the first location by two or more electrodes. In some embodiments, at least one of the two or more electrodes are not located at the first position. In some embodiments, the resonant response is detected at two or more electrodes of the at least one electrode.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

### Brief Description of Drawings

Embodiments of the present disclosure will now be described by way of non-limiting examples with reference to the drawings, in which:
Figure 1 is a graph illustrating resonance from a neural structure responsive to a deep brain stimulation (DBS) signal;
Figure 2 is a graph illustrating resonance from a neural structure responsive to a patterned DBS signal;
Figure 3 graphically illustrates observed resonance from a neural structure responsive to patterned stimulation at 0.3mA, 0.67mA, 1.5mA, and 3.38mA at different depths of insertion of an electrode lead proximate to the subthalamic nucleus of a patient;
Figure 4a is a graph illustrating evoked resonances beginning to diverge into two peaks in response to patterned DBS with an amplitude of 1.5mA;
Figure 4b is a graph illustrating evoked resonances diverging into two peaks in response to patterned DBS with an amplitude of 2.25mA;
Figure 4c is a graph illustrating two separate evoked resonant peaks in response to patterned DBS with an amplitude of 3.375mA;
Figure 5 graphically illustrates observed resonance from a neural structure responsive to patterned stimulation at 0.3mA, 0.67mA, 1.5mA, and 3.38mA at different depths of insertion of an electrode lead proximate to the subthalamic nucleus of a patient;
Figure 6 graphically illustrates observed resonance from a neural structure responsive to patterned stimulation at 0.3mA, 0.67mA, 1.5mA, and 3.38mA at different depths of insertion of an electrode lead proximate to the subthalamic nucleus of a patient;
Figure 7 graphically illustrates observed resonance from a neural structure responsive to patterned stimulation at 0.3mA, 0.67mA, 1.5mA, and 3.38mA at different depths of insertion of an electrode lead proximate to the subthalamic nucleus of a patient;
Figure 7A graphically illustrates observed resonance from a neural structure responsive to patterned stimulation at 1.5mA at different depths of insertion of an electrode lead proximate to the subthalamic nucleus of a patient and an associated spectrogram based on a wavelet transform for each insertion depth;
Figure 7B graphically illustrates observed resonance from a neural structure responsive to patterned stimulation at 3.38mA at different depths of insertion of an electrode lead proximate to the subthalamic nucleus of a patient and an associated spectrogram based on a wavelet transform for each insertion depth;
Figure 8 is a schematic illustration of an electrode lead tip for implantation in a brain;
Figure 9 is a schematic illustration of an electrode lead implanted in the subthalamic nucleus of a brain;
Figure 10 is a schematic illustration of a system for administering DBS;
Figure 11 is a flow diagram illustrating a process for locating a DBS electrode in the brain;
Figure 12 is a flow diagram illustrating a process for monitoring and processing resonant responses at multiple electrodes in response to stimulation at multiple electrodes;

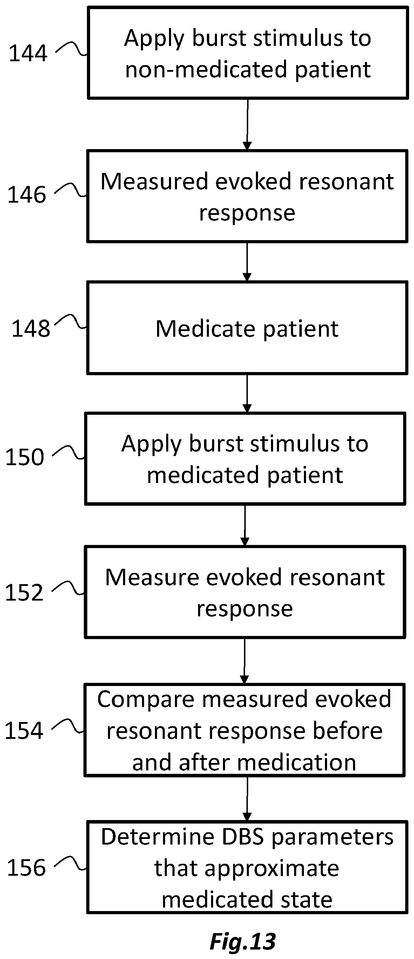

Figure 13 is a graphical illustration of resonant responses measured at different electrodes implanted in a brain responsive to a stimulation signal in accordance with the process shown in Figure 12;
Figure 14 graphically illustrates switching between periods of therapeutic and non-therapeutic stimulation relative to a resonant activity feature of an evoked response;
Figure 15a illustrates a patterned stimulation signal according to an embodiment of the present disclosure;
Figure 15b illustrates another patterned stimulation signal according to an embodiment of the present disclosure
Figure 16 is a three-dimensional reconstructions of an electrode array implanted in a STN (smaller mass) and sub anti a nigra .(larger mass) of a patient;
Figures 17A to 17C is a merged MRI and CT scan of a patient's brain showing the positioning of an electrode array;
Figure 18 graphically illustrates the variation in ERNA amplitude relative to electrode position;
Figure 19A graphically illustrates ERNA frequency vs DBS amplitude for 19 brain hemispheres;
Figure 19B graphically illustrates ERNA amplitude vs DBS amplitude for 19 brain hemispheres;
Figure 19C graphically illustrates Relative beta (RMS_{13-30 Hz}/RMS_{4-45 Hz}) vs DBS amplitude for 19 brain hemispheres;
Figure 19D graphically illustrates Relative beta correlated with ERNA frequency (ρ = 0.601, p < 0.001) for 19 brain hemispheres;
Figure 20A graphically illustrates ERNA frequency washout over consecutive 15 s periods post-DBS and in the last 15 s pre-DBS for 19 brain hemispheres;
Figure 20B graphically illustrates ERNA amplitude washout for 19 brain hemispheres;
Figure 20C graphically illustrates Relative beta washout for 19 brain hemispheres; and
Figure 20D graphically illustrates Washout in the 200-400 Hz HFO band for 19 brain hemispheres.

### Description of Embodiments

Embodiments of the present disclosure relate to improvements in neuro-stimulation in the brain. Specifically, embodiments of the present disclosure improve upon techniques for placement of DBS electrodes in the brain by comparing evoked resonant neural activity (ERNA) responsive to electrical stimuli applied to the DBS electrodes.

DBS devices typically apply a constant amplitude stimulus to a target area of the brain at a constant frequency of 130 Hz. It has previously been determined that application of such a stimulus evokes a neural response from the target area of the brain, and that the neural response comprises a resonant component. It has previously been reported that continuous DBS at conventional frequencies does not allow a long enough time window to observe the resonant activity. However, by monitoring evoked neural responses in the brain after stimulation has ceased (by patterning the stimulation signal or otherwise), the resonant activity can be monitored. This is described in detail in international application number PCT/AU2017/050809 Such techniques have applications both for reducing the physical effects associated with motor diseases, and also the detrimental effects of other neurological conditions, neuropsychiatric disorders, sensory disorders, and pain.

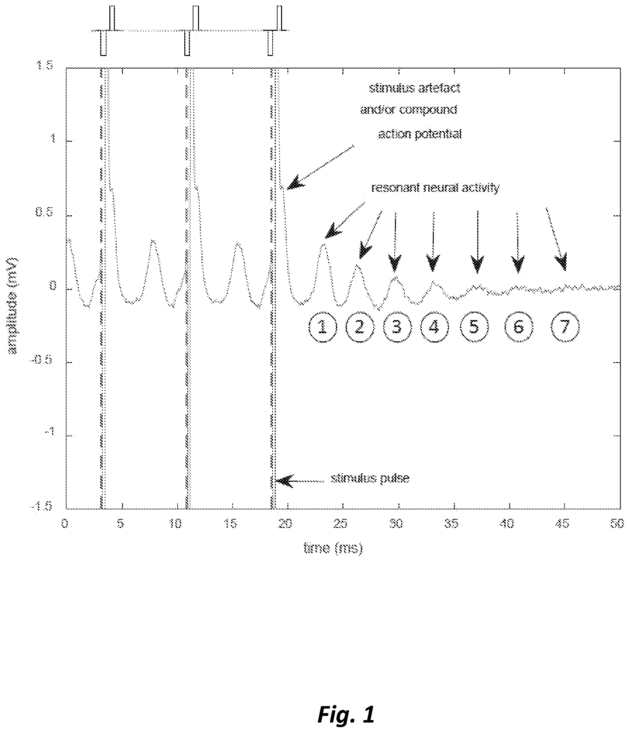

Figure 1 graphically illustrates a response from a neural circuit stimulated by a 130 Hz signal delivered from a neurostimulator via an electrode lead, such as the 3387 electrode lead manufactured by Medtronic (RTM), implanted in the subthalamic nucleus (STN) of a Parkinson's disease (PD) patient. Each response to a stimulus pulse comprises an evoked compound action potential (ECAP) component together with a component of evoked resonant neural activity (ERNA) occurring after the ECAP. The ECAP typically occurs within 1-2 milliseconds of the stimulus pulse. The graph shows the response to the last three consecutive pulses of a 60 second period of continuous stimulation followed by a period of no stimulation. It can be seen that the evoked resonant response to each of the first two stimulus pulses shown in Figure 1 is cut short by the onset of the next stimulus pulse, such that only a single secondary peak is detected. However, the evoked resonant response to the third (and final) pulse is able to resonate for longer and so can be clearly seen in the form of a decaying oscillation with at least seven peaks for a post-stimulus period of about 30 milliseconds.

It has been found that by controlling DBS parameters in certain ways, a non-therapeutic stimulus can be administered which evokes a resonant neural response (ERNA) in a patient without having any therapeutic impact or causing undesirable side effects. Such non-therapeutic stimuli can be used to reliably measure ERNA without causing sustained changes to the resonant neural circuit or the patient's symptomatic state. Non-therapeutic stimulation is preferably achieved by administering a stimulus comprising a short burst of pulses followed by a period of no stimulation, and the ERNA is measured during this period of no stimulation. By doing so, the total charge or energy provided to the patient is below a therapeutic threshold, and the measured ERNA provides information concerning the patient's natural state (without therapy). In an alternative embodiment, the overall charge or energy provided to the patient may be reduced by reducing the amplitude of the stimulation signal below a therapeutic threshold. However, doing so may also reduce the amplitude of peaks in the ERNA making it more difficult to observe.

Patterned stimulation can also be used to monitor and analyse evoked resonant neural activity during therapeutic stimulation of a patient. By patterning the stimulation signal, therapeutic stimulation can be maintained whilst providing time windows in which to monitor resonant responses past that of the first resonant peak or more preferably past two or more resonant peaks.

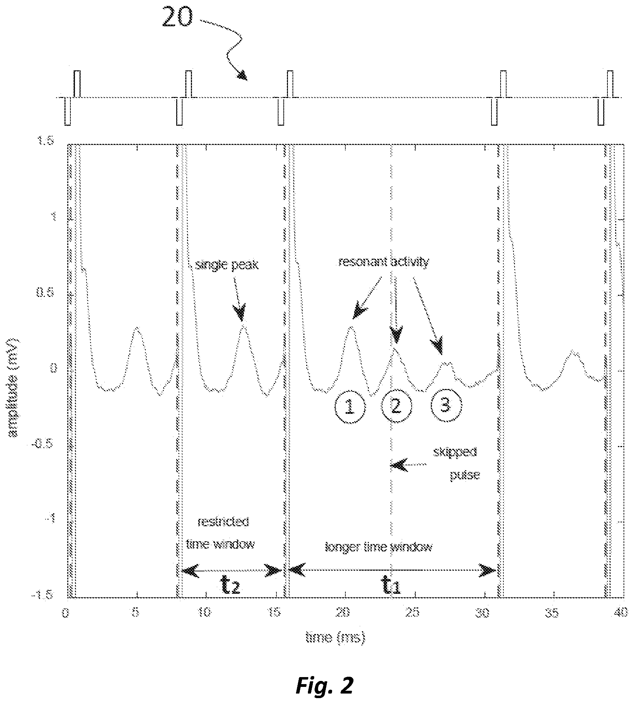

Figure 2 graphically illustrates an example therapeutic patterned DBS stimulus 20 and the associated evoked resonant response according to an embodiment of the present disclosure. The patterned stimulus 20 is shown above the graph to illustrate the correlation between stimulus and response. In the patterned stimulus, a single pulse has been omitted from an otherwise continuous 130 Hz pulse train. The pulse train therefore includes a plurality of bursts of pulses of continuous stimulation, each burst separated by a first time period t₁, each of the plurality of pulses separated by a second time period t₂. Continuation of the stimulus before and after omission of a pulse (or more than one pulse) maintains the therapeutic nature of the DBS, whilst the omission of a pulse allows for resonance of the ERNA to be monitored over several (3 in this example) resonant cycles before the next stimulation pulse interrupts this resonance.

In summary, by patterning non-therapeutic and therapeutic stimuli, an evoked response can be monitored over a longer period of time than with conventional non-patterned stimulation. Accordingly, stimuli are preferably applied in bursts of multiple pulses, each burst separated by a first time period t₁ of no stimulation, each pulse separated by a second time period t₂. For example, a stimulus signal may comprise a series of 10-pulse bursts at 130 Hz. To increase repeatability of results, the multi-pulse burst may be repeated after a predetermined period of no stimulation. For example, the multi-pulse burst may be repeated each second. The duration of the first time period t₁ is greater than that of the second time period t₂. The ratio between the duration of the burst and the duration between bursts may be chosen so as to ensure that relevant properties of the ERNA can be monitored easily and efficiently. In some embodiments, the duration of each burst is chosen to be between 1% and 20% of the duration of no stimulation between bursts.

Patterned stimulation may also be implemented by interleaving therapeutic stimulation (e.g. at a frequency of 130 Hz) with bursts of stimulation having a lower frequency (e.g. 90 Hz). The frequency of these interleaving bursts is preferably low enough to allow for multiple ERNA peaks to be observed. Equally, the frequency of these interleaving bursts is preferably high enough to be within the therapeutic frequency range for DBS. The transition between frequency may be abrupt or, alternatively the change in frequency may be gradual. Applying ramps to the frequency of the pulses to avoid an abrupt step change in frequency may be advantageous.

Additionally or alternatively to adjusting the frequency of the applied stimulus, the amplitude of pulses may be modulated over time. This may include applying a ramp to increase the pulse amplitudes over several pulses within a burst and/or a ramp to decrease the pulse amplitudes over several pulses within a burst. To enhance the monitoring of ERNA it may be advantageous to apply a fixed amplitude to the pulses preceding the observation window, and if this amplitude differs from that applied at other times (e.g. to maximise therapeutic benefit), then applying ramps to the amplitude of the pulses to avoid an abrupt step change in amplitude may be advantageous.

Using patterning techniques such as those described above, ERNA can be monitored in response to stimuli having differing amplitudes. From these observations, it has been found that a relationship exists between the effectiveness of therapeutic stimulation and the change in common waveform characteristics of evoked resonance responsive to stimuli having differing amplitudes. For example, for electrodes which administer effective therapeutic stimulation, increasing stimulation amplitude leads to a change in amplitude of evoked resonance. In contrast, for electrodes which are less effective for therapy, a change in stimulation amplitude does not substantially affect the amplitude of evoked resonance.

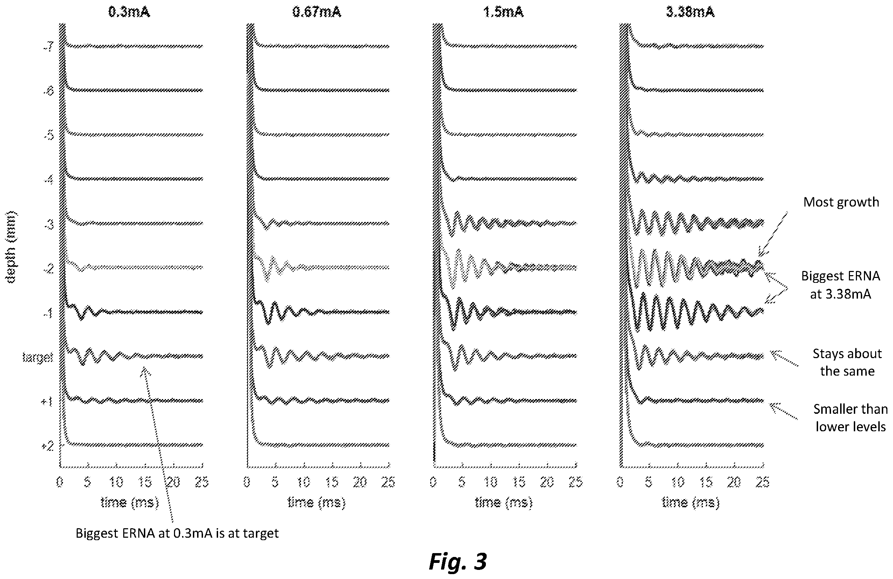

Figure 3 graphically illustrates observed ERNA responsive to patterned stimulation at 0.3mA, 0.67mA, 1.5mA, and 3.38mA (from left to right) at different depths of insertion of an electrode lead proximate to the subthalamic nucleus of a patient. Stimulation was applied to a single electrode in bursts of 10 pulses at 130Hz. For each amplitude, 2 bursts were applied per second for 4 seconds each (i.e. 8 bursts per amplitude). ERNA responses were extracted and detrended by filtering each response using a high-pass filter (3^{rd} order Butterworth, fc = 130Hz) and a low-pass filter (1^{st} order Butterworth, fc = 1000Hz). The depth scale on the x-axis of each graph in Figure 3 represents a position of the electrode relative to a surgical target. It is noted that this target does not necessarily correspond to an ideal electrode location, but rather a fixed reference location in the patient's brain that provides the surgeons with a location for which to aim during implantation of the electrode lead. For surgical implantation the surgical target is typically chosen based on anatomical landmarks identified from stereotactic imaging or the like and is typically around 2 mm to 4 mm deeper than the therapeutic target.

Referring to the left most plot in Figure 3 pertaining to ERNA responsive to stimulation at 0.3mA amplitude, it can be seen that the largest amplitude of ERNA appears at the surgical target, i.e. a depth of 0. However, as the stimulation amplitude increases, it can be seen that the amplitude of the ERNA measured at the surgical target remains substantially unchanged. In contrast, the amplitude of observed ERNA at -1, -2, and -3 mm (i.e. 1, 2, and 3 mm above the surgical target) increases substantially, the largest change being at a depth of -2 (i.e. 2 mm from the surgical target). It is thought that therapeutic DBS may be most effective at this location.

The above shows that by observing ERNA responsive to stimulation applied at both different implantation locations and different amplitudes, optimal electrode positioning for therapeutic stimulation may be more efficiently and more accurately realised.

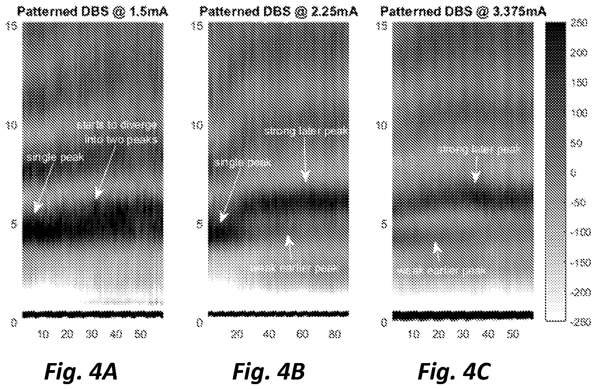

It has also previously been found that in some instances evoked activity comprises multiple resonances. Figures 4a, 4b and 4c illustrates ERNA in response to continuous DBS at 1.5 mA, 2.25 mA and 3.375 mA respectively. At 1.5 mA the resonant ERNA starts as a single peak, which can be seen to begin to diverge slightly into two peaks. At 2.25 mA, the dominance switches to the later of the two peaks. However, the earlier peak, which was dominant at 1.5 mA, continues at a lower amplitude. At 3.375 mA, two peaks are present, with the later peak dominating. It is thought that these multiple resonant peaks correspond to activity in different neural circuits. The relative amplitude between these resonant responses (or other features, such as temporal or spectral properties) may be an indicator of therapeutic state.

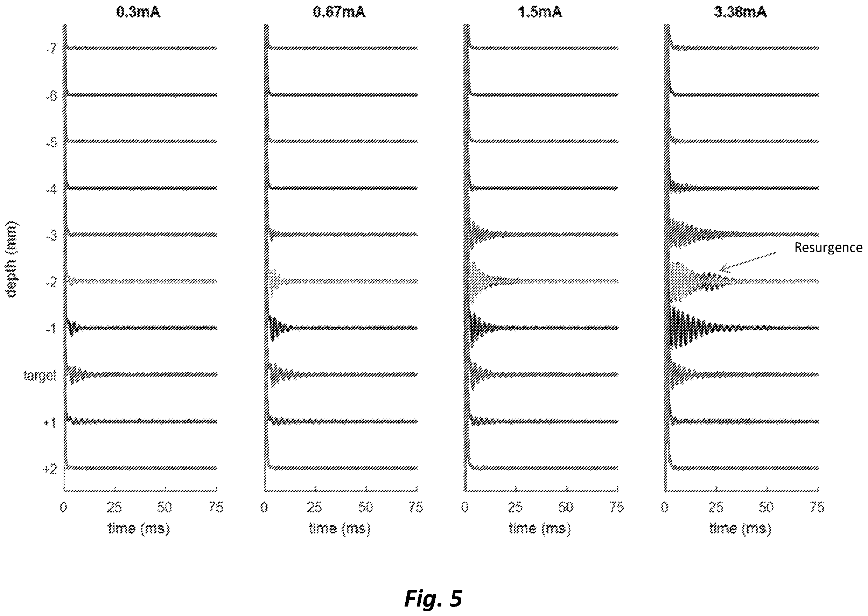

It is hypothesised that resurgence in ERNA is the result of the superposition of multiple resonant responses. Figure 5 graphically illustrates the observed ERNA responsive to the same patterned stimulation as that described above with reference to Figure 3 at 0.3mA, 0.67mA, 1.5mA, and 3.38mA (from left to right) at different depths of insertion of an electrode lead proximate to the subthalamic nucleus of a patient. Figure 5 shows the observed ERNA over a longer time period (75 ms). Resurgence can be seen in the ERNA observed at -2 mm depth responsive to stimulation at 3.38 mA.

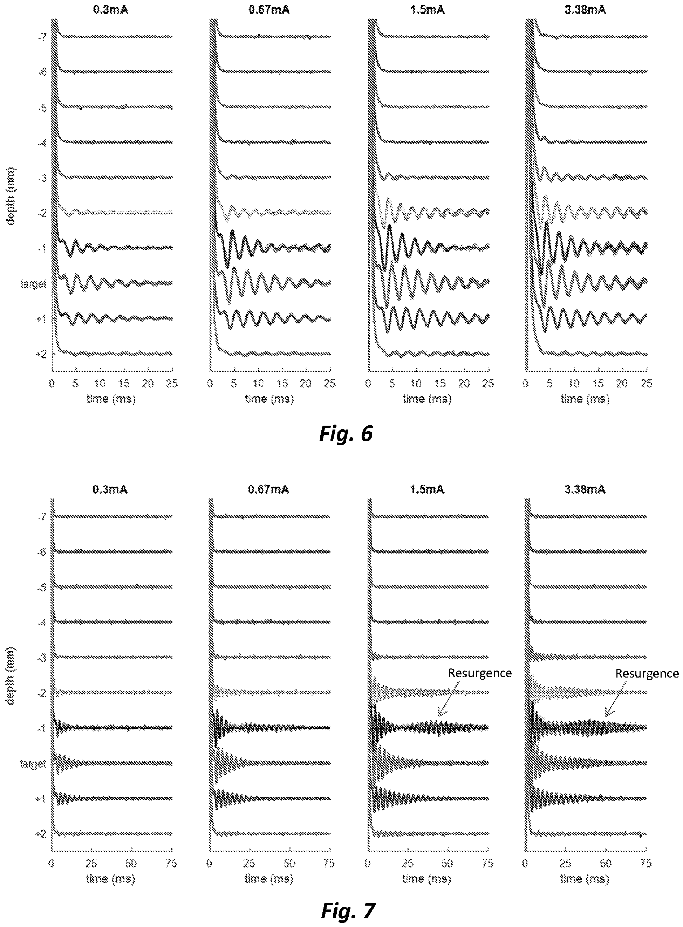

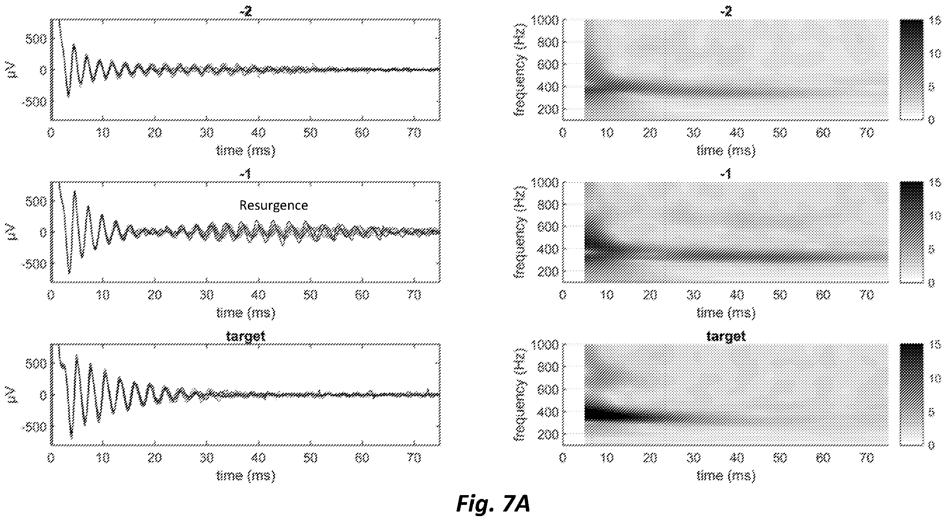

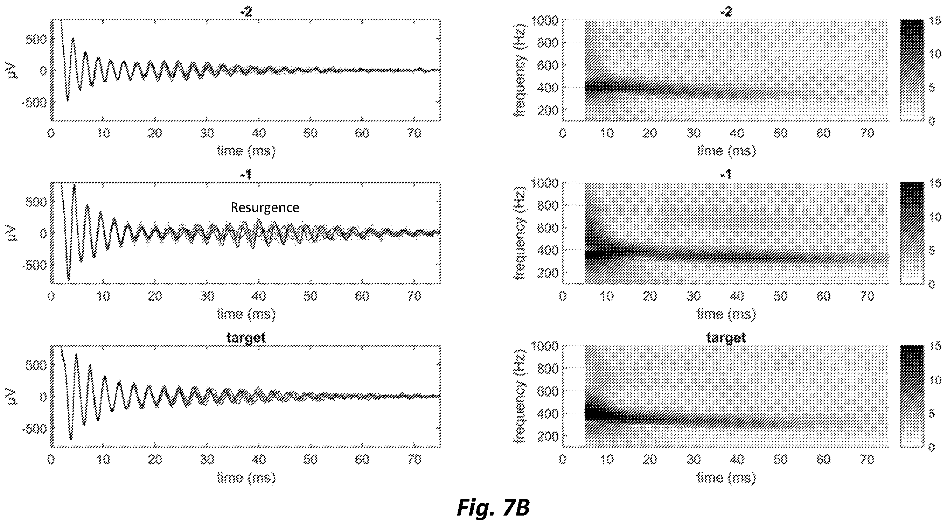

Figures 6 and 7 graphically illustrate observed ERNA for another patient responsive to the same patterned stimulation as that described above with reference to Figures 3 and 5. Figure 6 shows ERNA over 25 ms whereas Figure 7 shows ERNA over a longer time period of 75 ms. For this patient, over the shorter time period of 25 ms shown more clearly in Figure 6, the growth in ERNA amplitude at surgical target and -1 mm are comparable. However, over the longer period of 75 ms shown in Figure 7, substantial resurgence is observed at -1 mm which is thought to correspond with optimal electrode positioning for therapeutic stimulation. Thus, it seems that a correlation exists between resurgence in ERNA and optimal electrode placement.

Figures 7A and 7B illustrates the observed ERNA (left) shown in Figure 7 for stimulation amplitudes of 1.5 mA and 3.38 mA respectively for depths of -2 mm, -1 mm from the surgical target and at the surgical target. On the right of each of Figures 7A and 7B, an associated spectrogram based on a wavelet transform corresponding to the ERNA depicted on the left is provided for each depth. Each spectrogram represents an average of the spectra of 8 responses to 8 bursts of stimuli (as noted above). The spectrograms were generated using the MatLab (RTM) cwt function ('morse' wavelet, gamma=5, time-bandwidth=120), 32 voices per octave).

The wavelet spectrograms support the hypothesis that the resurgence is consistent with superposition or resonances at two frequencies. For example, the wavelet spectrogram for the observed ERNA at -1 mm insertion depth appear to comprise two frequency components between 0 and around 20 ms. From around 15 ms, the frequency components appear to merge into a single frequency component. Resurgence of the observed ERNA appears to occur just after this merging of the multiple frequency components.

It can also be seen from the observed ERNA plots in Figures 7A to 7B that latency between stimulation and onset of observed ERNA varies with insertion depth. Specifically, latency appears to increase the closer the electrode is positioned to the surgical target. It will therefore be appreciated that ERNA onset latency may be used to determine a position of an electrode to a surgical target, a distance to the target, a direction to the target (triangulating using multiple (i.e. three or more) electrodes) etc.

It will be appreciated that the various characteristics of the ERNA waveform such as time to first peak, delays between ERNA peaks, variable ERNA amplitude and resurgence are likely to be patient-specific. Advantageously, any reliance on such data for controlling DBS stimulation will be based on an initial characterisation of ERNA waveforms and movement impairment associated with each patient in order to generate a regime for stimulation control (e.g. closed loop control).

The identification of a correlation between changes in resonant behaviour of stimulated neural circuits and a patient's disease symptoms present several opportunities to improve aspects of DBS therapy, including but not limited to techniques for initial implantation and subsequent repositioning of DBS electrodes, together with techniques for setting parameters of DBS stimulation and using feedback to adjust DBS parameters in real time whilst DBS therapy is underway.

A number of practical applications of the above described evoked resonant neural activity will now be discussed with reference to several embodiments. In the embodiments, one or more electrode leads may be used for stimulation of one or more neural structures within one or both hemispheres of the brain, each lead comprising one or more electrodes located near the tip of each lead. Each of the electrodes may be used for stimulation, monitoring, or both stimulation and monitoring. One or more of these electrodes may be implanted. Implanted electrodes may be used independently or in addition to one or more electrodes placed on the outside of the brain or skull.

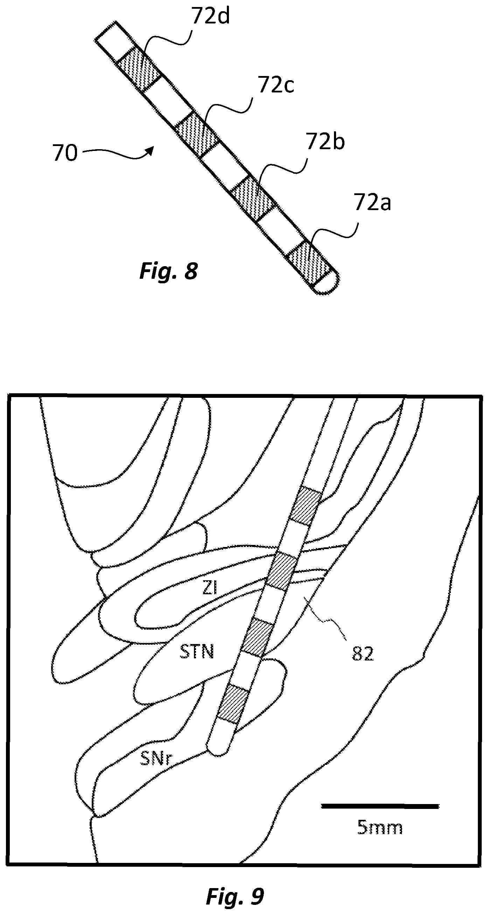

A typical DBS electrode lead tip 70, such as that incorporated into the Medtronic (RTM) DBS Lead Model 3387, is shown in Figure 8. The lead tip 70 comprises a first electrode 72a, a second electrode 72b, a third electrode 72c, and a fourth electrode 72d. Once implanted into the brain, each of the electrodes 72a, 72b, 72c, 72d may be used to apply a stimulus to one or more neural structures or monitor and optionally record the evoked response (including ERNA) from neural circuits to the stimulus. In other embodiments, leads with more electrodes or electrodes with different sizes or topologies may be used. In addition, one or more reference electrodes may be located at a remote site and used to complete the electrical circuit when one or more electrodes on the DBS lead are activated for stimulation or used for signal monitoring.

The target location for the lead tip 70 varies dependent on the neural structure. Example target structures include but are not limited to the subthalamic nucleus (STN), the substantia nigra pars reticulata (SNr), and the globus pallidus interna (GPi).

Figure 9 shows the lead tip 70 implanted into a brain at a target structure, in this case the subthalamic nucleus (STN) 82. It will be appreciated that intersecting the electrode tip 70 with the subthalamic nucleus (STN) 82, which has a typical diameter of 5 to 6 mm, can be a very difficult surgical task. Techniques such as stereotactic imaging, microelectrode recordings, intraoperative x-ray imaging, and applying therapeutic stimulation whilst monitoring patient symptoms, are currently used to localise the electrode tip 70. However, these methods can lack accuracy. Additionally, existing methods usually require the patient to be awake for the procedure, since voluntary responses from the patient can be used to confirm that the electrode is at a suitable location relative to a target structure in the brain. For this reason, many potential recipients of DBS therapy turn down the option because they are not comfortable with having to be awake during the surgical procedure.

The accuracy of locating electrodes of the electrode tip 70 within a target structure can be greatly increased by using a series of patterned stimulations to generate and measure an evoked resonant response from a neural target. Such techniques can obviate the need for the patient to be awake during the implantation procedure, since an electrode can be located much more accurately at the correct location within the brain and relative to a target neural structure. This means that patients can be under sedation or general anaesthetic during the surgery since no patient feedback is required to locate the electrode to a satisfactory degree of accuracy.

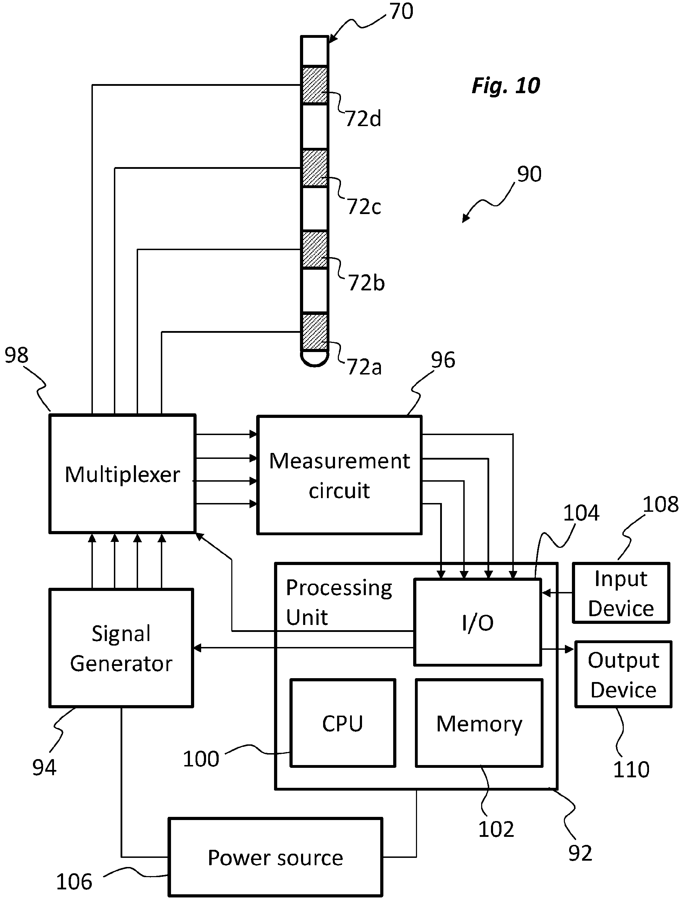

An example DBS delivery system 90 according to an embodiment of the present disclosure is illustrated in Figure 10. The system 90 comprises the lead tip 70 of Figure 8 including the plurality of integrated electrodes 72a, 72b, 72c, 72d, together with a processing unit 92, a signal generator 94, a measurement circuit 96 and an optional multiplexer 98. The processing unit comprises a central processing unit (CPU) 100, memory 102, and an input/output (I/O) bus 104 communicatively coupled with one or more of the CPU 100 and memory 102.

In some embodiments, the multiplexer 98 is provided to control whether the electrodes 72a, 72b, 72c, 72d are connected to the signal generator 94 and/or to the measurement circuit 96. In other embodiments the multiplexer may not be required. For example, the electrodes 72a, 72b, 72c, 72d may instead be connected directly to both the signal generator 94 and the measurement circuit 96. Although in Figure 10 all of the electrodes 72a, 72b, 72c, 72d are connected to the multiplexer 98, in other embodiments, only one or some of the electrodes 72a, 72b, 72c, 72d may be connected.

The measurement circuit 96 may include one or more amplifiers and digital signal processing circuitry including but not limited to sampling circuits for measuring neural responses to stimulation, including ERNA. In some embodiments the measurement circuit 96 may also be configured to extract other information from received signals, including local field potentials. The measurement circuit 96 may also be used in conjunction with the signal generator 94 to measure electrode impedances. The measurement circuit 96 may be external to or integrated within the processing unit 92. Communication between the measurement circuit 96 and/or the signal generator 94 on the one hand and the I/O port on the other may be wired or may be via a wireless link, such as over inductive coupling, WiFi (RTM), Bluetooth (RTM) or the like. Power may be supplied to the system 90 via at least one power source 106. The power source 106 may comprise a battery such that elements of the system 90 can maintain power when implanted into a patient.

The signal generator 94 is coupled via the multiplexer 98 to one or more of the electrodes 72a, 72b, 72c, 72d and is operable to deliver electrical stimuli to respective electrodes based on signals received from the processing unit 92. To this end, the signal generator 94, the multiplexer 98 and the processing unit 92 are also communicatively coupled such that information can be transferred therebetween. Whilst the signal generator 94, multiplexer 98, and the processing unit 92 in Figure 10 are shown as separate units, in other embodiments the signal generator 94 and multiplexer may be integrated into the processing unit 92. Furthermore, either unit may be implanted or located outside the patient's body.

The system 90 may further comprise one or more input devices 108 and one or more output devices 110. Input devices 108 may include but are not limited to one or more of a keyboard, mouse, touchpad and touchscreen. Examples of output devices include displays, touchscreens, light indicators (LEDs), sound generators and haptic generators. Input and/or output devices 108, 110 may be configured to provide feedback (e.g. visual, auditory or haptic feedback) to a user related, for example, to characteristics of ERNA or subsequently derived indicators (such as proximity of the electrode 70 relative to neural structures in the brain. To this end, one or more of the input devices 108 may also be an output device 110, e.g. a touchscreen or haptic joystick. Input and output devices 108, 110 may also be wired or wirelessly connected to the processing unit 92. Input and output devices 108, 110 may be configured to provide the patient with control of the device (i.e. a patient controller) or to allow clinicians to program stimulation settings, and receive feedback of the effects of stimulation parameters on ERNA characteristics.

One or more elements of the system 90 may be portable. One or more elements may be implantable into the patient. In some embodiments, for example, the signal generator 94 and lead 70 may be implantable into the patient and the processing unit 92 may be external to the patient's skin and may be configured for wireless communication with the signal generator via RF transmission (e.g. induction, Bluetooth (RTM), etc.). In other embodiments, the processing unit 92, signal generator 94 and lead 70 may all be implanted within the patient's body. In any case, the signal generator 94 and/or the processing unit 92 may be configured to wirelessly communicate with a controller (not shown) located external to the patient's body.

One embodiment of the present disclosure provides a system and method for localising the lead tip 70 within a target structure of the brain using measured ERNA. During an operation for implantation of the lead tip 70 into the brain, instead of relying on low accuracy positioning techniques as described above to estimate the location of electrodes relative to neural structures within the brain, the system 90 may be used to provide real-time feedback to the surgeon based on characteristics such as the strength and quality of evoked response signals received from one or more electrodes of the lead tip 70. This feedback may be used to estimate position within the target structure in three dimensions and to inform the decision of whether to reposition the electrodes or remove and reimplant the electrodes along a different trajectory.

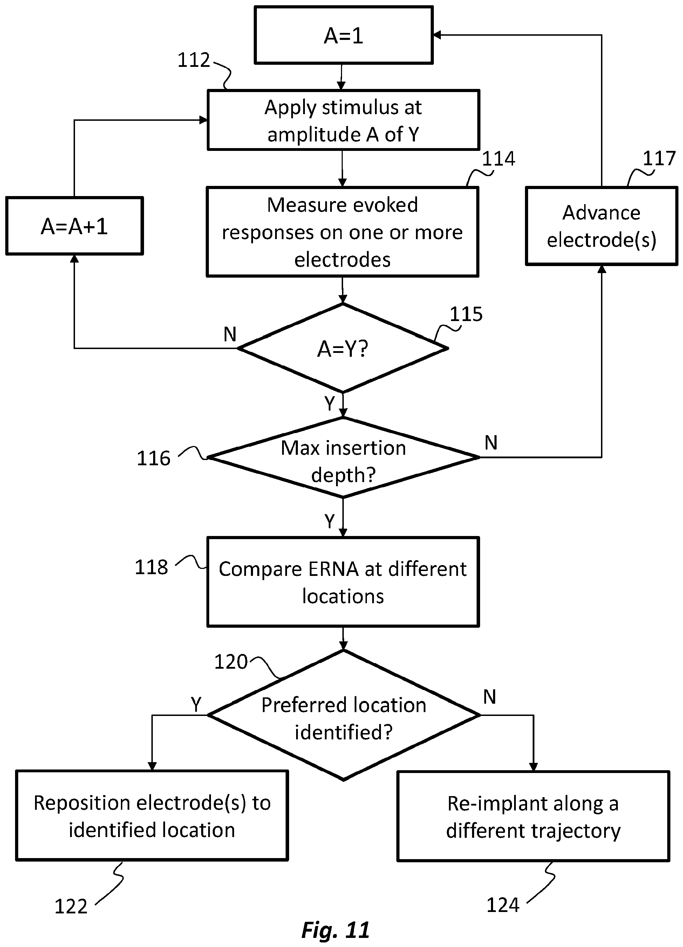

Figure 11 shows a general example of such a process in which ERNA is measured at various electrode locations in response to stimulation at various amplitudes. The process begins at step 112 with an electrode lead tip such as that described with reference to Figure 8 having being advanced during surgery towards a target neural structure along a predefined trajectory. The step size (or spatial resolution) by which the electrode lead is advanced may be chosen by the surgeons and/or clinicians. In some embodiments, the step size is 1 mm. At step 112, a patterned stimulus, such as those described above, is applied at the electrode at an amplitude A of Y amplitudes, where Y is the total number of different amplitudes to be used for stimulation, to an electrode of the lead tip 70. The stimulus may be applied for the whole time whilst the lead tip 70 is being implanted. Alternatively, the patterned signal may be repeated a predetermined number of times, such as 10 times. The evoked response may be measured at the same electrode as that used to apply the stimulus or may be measured at one or more different electrodes. By doing so, a more accurate estimate of the location of each electrode relative to the target neural structure may be provided.

After ERNA has been measured at step 114, at step 115 if ERNA has been measured for all Y amplitudes of patterned stimulation, the process continues to step 116. Otherwise, steps 112 and 114 are repeated with the electrode(s) in the same location at different amplitudes from A to Y. The size of amplitude increments may be chosen to provide the necessary granularity of results to allow accurate characterisation of the amplitude varying ERNA, which may itself be patient specific. In some embodiments, the stimulation amplitudes are 0.3 mA, 0.67 mA, 1.5 mA and 3.38 mA as shown in Figures 3 and 5 to 7.

At step 116, once stimuli at all Y amplitudes has been applied and respective ERNA recorded, the electrode lead is advanced at step 117. Steps 112 to 116 are then repeated until the electrode lead tip has been inserted to the maximum allowable depth, which may be in the target neural structure or slightly beyond it.

By repeating steps 112 to 116, a profile or map of evoked responses at different locations along the insertion trajectory may be generated for different amplitudes of stimulation. The profile of evoked responses may include measurements from multiple electrodes or from just one electrode. The profile of evoked responses at different depths and different amplitudes may be output to the one or more output devices 110. The profile of evoked responses may then be compared at step 118 in order to determine whether a preferred electrode location can be identified. The identification of preferred electrode location may be based on different ERNA features, including relative differences between or spatial derivatives of amplitude, rate of decay, rate of change, and frequency, at different insertion positions (e.g. the location that produces the largest resonances), in particular at different stimulation amplitudes.

The identification of a preferred electrode location may also be based on comparison with template ERNA activity, where the templates have been derived from recordings from other patients. The profile of evoked responses may also be used to estimate the trajectory of the electrode lead 70 through the target neural structure, including the boundaries of the structure and the region intersected (e.g. the trajectory passed through the medial or lateral region). The profile of evoked responses may also be used to estimate the proximity to the target structure, in the event that the target structure is not intersected by the insertion trajectory.

If at step 120 a preferred electrode location can be identified, the electrode lead tip 70 can be repositioned at step 122, such that an electrode is positioned at the preferred location. Alternatively, for embodiments that include electrode lead tips with a large number of electrodes, the electrode positioned closest to the preferred location can be nominated for subsequent use in applying therapeutic stimulation. If at step 120 a preferred location cannot be identified, the surgeon and/or clinician may choose to remove the electrode and re-implant along a different trajectory.

In some embodiments, the lead tip 70 used to determine optimum electrode placement (as described above) may be a temporary lead used only for such procedures. Accordingly, subsequent to identifying an optimum location for stimulation, the lead tip 70 may be removed and replaced with a permanent electrode or electrode array for long term DBS therapy.

Another embodiment of the present disclosure provides a system and method for determining the relative positions of an array of electrodes with respect to a target neural structure and then selecting a preferred electrode to use for applying therapeutic stimulation. This process could be performed during electrode implantation surgery to assist in the positioning of electrodes, or with previously implanted electrodes when programming the device to deliver therapeutic stimulation. A stimulus may be applied at more than one of the electrodes of the array, for example two or more of electrodes 72a, 72b, 72c, 72d in the case of electrode array 70. Where a patterned stimulation regime is used, sequential bursts of a stimulus pattern may be applied to different ones of the electrodes 72a, 72b, 72c, 72d. Alternatively, a full stimulus pattern may be applied at one electrode, followed by another full stimulus pattern at another electrode. By doing so, a determination may be made concerning which electrode of an electrode array is positioned best to provide therapeutic stimulation to one or more of the target neural structures; for example, which of the electrodes 72a, 72b, 72c, 72d is best positioned within a target neural structure.

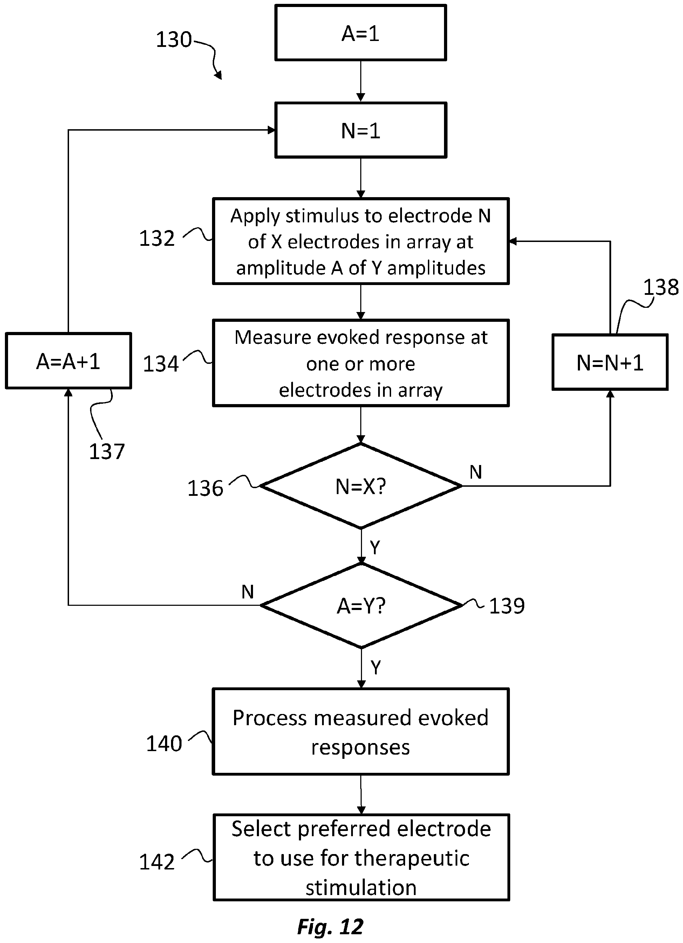

Figure 12 illustrates an example process 130 for measuring evoked response from an array of multiple electrodes. At step 132, a stimulus is applied to the first electrode of an array of X electrodes (e.g. electrode 72a in the case of the lead tip 70) at an amplitude A of Y different amplitudes. The stimulus applied may be a burst patterned stimulus as described above. The evoked response from a target neural structure is measured at one or more of the electrodes in the array at step 134. In the case of the lead tip 70, for example, the evoked response may be measured at the second, third and fourth electrodes 72b, 72c, 72d when the first electrode 72a is being stimulated. Additionally or alternatively, the evoked response may be measured at the same electrode as the electrode being stimulated. In some embodiments, the evoked responses received at one or more of the electrodes may be stored in memory. Once the evoked response has been measured at one or more electrodes, another electrode is selected for stimulation. This may be achieved by incrementing a counter, as shown in step 138, after the process 130 has checked at step 136 to see whether all electrodes in the array of X electrodes have been stimulated, i.e. whether the process has cycled through all of the electrodes in the system. If there are electrodes remaining to be stimulated, then the process repeats, applying a stimulus to the next selected electrode in the array. If all electrodes in the array have been stimulated and an evoked response to stimulation measured and recorded, another stimulation amplitude of the Y amplitudes may be selected for stimulation. This may be achieved, for example, by incrementing a counter as shown in step 137, after the process 130 has checked at step 139 to see whether all amplitudes Y have been cycled through. Steps 132 to 138 may then be repeated for the new stimulation amplitude A. If at step 139 it is found that all electrodes have been stimulated at all amplitudes, the resultant measured evoked responses are then processed at step 140.

Processing the evoked responses may involve comparing different ERNA features, including relative differences between or spatial derivatives of amplitude, rate of decay, rate of change, and frequency, across different combinations of electrodes used for stimulation and measurement and across each of the different stimulation amplitudes used for stimulation. For example, the processing may involve identifying the electrode that measures the largest change in evoked resonance amplitude in response to changes in stimulation condition (e.g. amplitude) or identifying the electrode with the largest resurgence or change in resurgence measured over increasing stimulation amplitudes. The identification of the preferred electrode location may also be based on a comparison with template ERNA activity. Templates may be derived from recordings from other patients or from one or more models or simulations.

Based on the processing of the evoked responses, a preferred electrode to use for therapeutic stimulation may be chosen at step 142. The results of the ERNA processing and a recommendation for the preferred electrode may be output to the one or more output devices 110. If the process has been performed during surgery, the results of the ERNA processing may also be used to determine which electrodes are within the target neural structure and whether to reposition the electrode array. The results may also be used to generate one or more templates for future processing of evoked responses in the same or different patients.

The evoked response may be measured at one or two or any number of electrodes in any configuration. For example, ERNA could be measured and/or recorded from different combinations of electrodes. Additionally or alternatively, measurement electrodes may implanted in and/or positioned external to the brain or skull.

The inventors have found that by comparing the measured evoked responses at each electrode in response to stimulation at another electrode, a determination can be made firstly of whether any of the electrodes are positioned within the target neural structure, secondly whether any of the electrodes are positioned at an optimum location within the target neural structure, and thirdly the direction and/or distance of a particular electrode from that target neural structure. In some embodiments, one or more of the presence, amplitude, natural frequency, damping, rate of change, envelope, resurgence and fine structure of a evoked resonant response (or superposition of resonant responses) to a stimulus may be used to identify the most effective electrode in an electrode array. Preferably, changes in one or more of these characteristics in response to patterned stimulation at differing amplitudes, may be used to identify the most effective electrode in an electrode array. Additionally, it can be seen that the evoked responses (and changes in characteristics of evoked responses in response to different stimulation amplitudes) vary depending on the position of the electrode used for stimulation, illustrating the feasibility of using the process illustrated in Figure 11 to localise electrodes within a target neural structure.

The process 130 of Figure 12 and other processes described herein may be performed by varying stimulation parameters other than amplitude (e.g. using different stimulation frequencies) or with more than one stimulating electrode in step 132 (e.g. stimulation applied concurrently through multiple electrodes on one or more electrode leads). For example, electrical stimuli may be directed to a target location in the brain by applying the stimuli from two or more spaced apart electrodes. Such electrodes may be positioned at locations proximate but not at the target location. Current steering may be utilised to steer the electrical stimuli to the target location or position in the brain so that the epicentre of the electrical stimuli is focused at the target location or position. To do so, an amplitude bias may be applied to one or more of the two or more spaced apart electrodes to steer current in a particular direction. Such current steering may be fixed or may be variable to enable two dimensional or three dimensional (with three or more spaced apart electrodes) shifting of focus of electrical stimuli.

The resonant response to the electrical stimuli may be measured at two or more spaced apart electrodes. In doing so, directional information may be obtained relating to the location or neural networks which may be the source of the response. The response characteristics obtained by the one or more electrodes may be used to aid current steering (e.g. setting the distribution of currents across electrodes that are active simultaneously so as to direct stimulation, therapeutic or otherwise to target locations) and the selection of active electrodes (e.g. which electrodes to use for stimulation). For example, as mentioned above, response characteristics can be used to estimate the spatial spread of activation relative to the target area. Using this information, the stimulation profile may be shaped using two or more electrodes to direct stimulation to particular areas of the brain, i.e. towards a target structure, and away from areas which the clinician does not wish to stimulate.

In a further embodiment, ERNA can also be used to optimize stimulation parameters used to target various medical conditions. For instance, once an electrode array such as the lead tip 70 has been accurately located within a target neural structure, the setting of stimulation parameters for therapeutic DBS can be aided by measuring ERNA, improving accuracy and time- and cost-efficiency, and reducing undesirable side-effects.

In addition to enhancing the accuracy of locating a DBS electrode in the brain, choosing electrode configurations for stimulation and optimising stimulation parameters, ERNA may be used to generate feedback for controlling the stimulation of electrodes. In some embodiments, feedback may be implemented using the system 90 shown in Figure 10.

In one embodiment, the system 90 may use a waveform template corresponding to a preferred patient state. The template may be generated using previous recordings of ERNA in a patient with reduced symptoms. For example, ERNA templates recorded from a medicated patient or a patient receiving effective stimulation treatment may be used. Alternatively, ERNA templates recorded from a healthy patient, e.g. a patient without a movement disorder, may be used. Templates may be constructed from the average of many recordings from one patient or several patients. In some embodiments, selected features of the ERNA waveform may be used instead of a complete template. For example, parameters of the ERNA such as the dominant frequency and amplitude components and/or temporal features may be used to enable improved electrode placement and control of therapeutic stimulation. In some embodiments, preferred ranges for different ERNA characteristics may be defined (e.g. stimulation is controlled such that the ERNA frequency remains within 250-270Hz).

In some embodiments, bursts of stimulation, such as those described above, in combination with the monitoring of ERNA may be used to identify a therapeutic resonant state (e.g. a state which correlates with good symptom suppression with minimal side effects and/or minimum electrical power consumption). From this information, therapeutic stimulation parameters required to produce the preferred therapeutic state may be identified. In some embodiments, these stimulation parameters may be used to apply continuous therapeutic DBS to the target neural structure.

Probe bursts for identifying resonant activity can be interleaved with the therapeutic DBS to re-assess the resonant state. These probe bursts may be implemented on a periodic basis, for example, every 10 seconds. In one embodiment, every 10 seconds, a probe burst may be applied for 1 second (e.g. 10 pulses at 130 Hz) and the ERNA assessed. The therapeutic stimulation parameters may then be adjusted or maintained based on the ERNA. For example, if there is a change in ERNA relative to the last probe burst, the stimulation parameters may be adjusted such that the ERNA becomes comparable with the previously measured ERNA and/or the template ERNA and/or an ERNA characteristic is within a desired range.

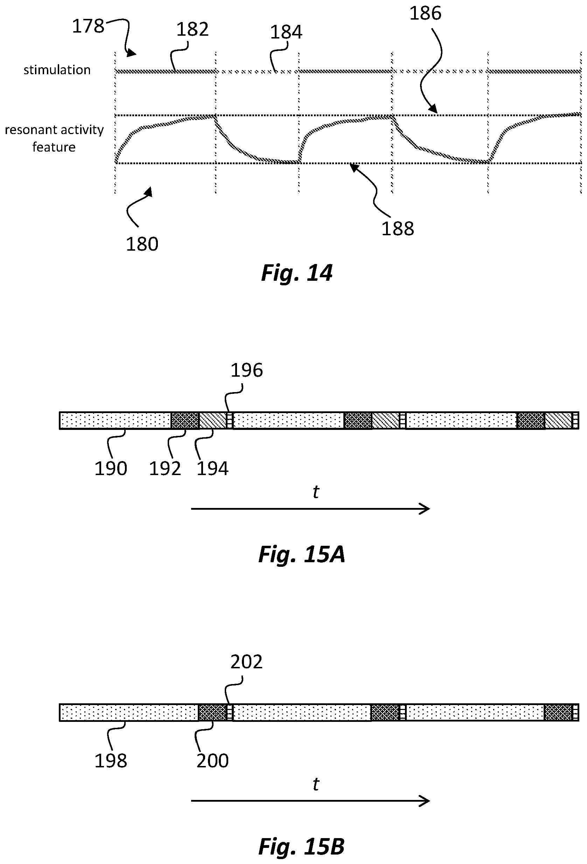

Figure 14 graphically compares a stimulation regime 178 comprising a patterned therapeutic stimulation signal 182 followed by a non-therapeutic patterned stimulation signal (comprising one or more bursts of pulses) 184 and a corresponding characteristic (e.g. resonant frequency) 180 of the ERNA varying between a preferred state 186 and a less than preferable state 188.

There are several different ways of implementing the patterned signals of embodiments described herein. Figures 15a and 15b illustrate two exemplary patterning profiles. In Figure 15a, the patterned profile includes a period of no stimulation 192 after a continuous stimulation block 190, followed by a burst of pulses 194 and another period of no stimulation 196. During the period of no stimulation, the ERNA may be measured and therapeutic stimulation signal adjusted (if required).

In an alternative embodiment, the system may monitor the ERNA after a final pulse of continuous stimulation 198 as shown in Figure 15b. After a period of monitoring 200, the therapeutic stimulation may then be adjusted for a period 202 after which the therapeutic stimulation 198 may be applied with the adjusted parameters. This regime may also be considered as continuous stimulation with periodic missing pulses. To this end, the continuous stimulation may be considered as a burst of pulses, and the period of no stimulation may be considered as the first time period t₁ as described with reference to Figure 2 above.

In other embodiments, instead of omitting stimulation during the monitoring period 200, stimulation may be maintained but with altered parameters. For example, conventional therapeutic stimulation at a frequency of, e.g. 130 Hz, may be applied during the therapeutic stimulation period 198. Then during the monitoring period 200, a stimulus having a different frequency, may be applied. The stimulus applied during the monitoring period may be lower than that applied during the stimulation period. For example, the stimulation frequency during this period may be in the region of 90 Hz. The frequency of this stimulus during the monitoring period 200 may be low enough to allow for multiple ERNA peaks to be observed. Equally, the frequency of the stimulus applied during the monitoring period 200 may be high enough to be within the therapeutic frequency range for DBS. As mentioned above, the transition between frequency may be abrupt or, alternatively the change in frequency may be gradual. Applying ramps to the frequency of the pulses to avoid an abrupt step change in frequency may be advantageous.

Additionally or alternatively, the amplitude of the stimulus applied during the monitoring period 200 may differ to that applied during the therapeutic period 198. For example, the amplitude of the stimulus applied during the monitoring period 200 may be less than that applied during therapeutic stimulation 198. An amplitude ramp may be applied to transition the stimulus between the therapeutic period 198 and the monitoring period 200 over several pulses, to avoid an abrupt step change in amplitude.

In some embodiments, characteristics of the stimulus other than amplitude and frequency may differ between the stimulation period 198 and the monitoring period. Examples of such characteristics include, but are not limited to, frequency, amplitude, pulse-width, net charge, electrode configuration, or morphology of the stimulus.

Whilst in embodiments described above, a single electrode array is used both to stimulate and record an evoked neural response, in other embodiments, electrodes may be distributed on multiple probes or leads in one or more target structures in either or both brain hemispheres. Equally, electrodes either implanted or positioned external to the brain may be used to stimulate or record or both stimulate and record an evoked neural response. In some embodiments, a combination of both microelectrode and macroelectrodes may be used in any foreseeable manner.

In a further application of the embodiments of the present invention ERNA measurements may be recorded and tracked over time to monitor the progression or remission of a disease or syndrome, or used as a diagnostic tool (e.g. to classify the patient's neurological condition). Such embodiments may also be used to provide medical alerts to the patient, a caregiver or a clinician in the event that the patient's state (as determined by ERNA) deteriorates towards an undesirable or critical state (e.g. a Parkinsonian crisis).

In yet another application, ERNA may be used to monitor the effects of medication over time, including the effects of adjusting medication doses, etc. Such an embodiment may also be used to provide medication alerts to the patient to remind them when a dose is required or when a dose has been skipped. Tracking medication effects with ERNA may also provide clinicians with information regarding whether medication is being taken as prescribed or whether medication is becoming less effective and requires dosing adjustment.

### Further analysis of ERNA and explanation of results

### DBS evokes resonant neural activity

The neural activity resulting from DBS pulses was investigated to determine if there were evoked responses that could feasibly be used as a biomarker. To preserve evoked activity we used a wide recording bandwidth, as well as symmetric biphasic pulses for stimulation, rather than conventional asymmetric pulses with a very long second phase, to minimize the temporal duration of stimulation artefacts.

Recordings were made from DBS electrodes immediately following their implantation in the STN of patients with PD who were still awake on the operating table , as PD is the predominant application for DBS. Furthermore, the STN's roles in regulation of motor, limbic, and associative function make it a neural target relevant to a number of different applications, including DBS treatment of dystonia, essential tremor, epilepsy, and obsessive-compulsive disorder.

It was found that STN-DBS evokes a large peak typically around 4 ms after each pulse. By examining the activity following the last pulse prior to cessation of DBS it was discovered that this peak is the first in a series with progressively decreasing amplitude. As this response has a form that resembles a decaying oscillation, we describe it as evoked resonant neural activity (ERNA).

To further investigate ERNA, we temporally patterned standard 130 Hz DBS to allow multiple peaks to be observed. We employed two novel patterns: skipping one pulse every second, and applying a burst often pulses every second. The 'skipped-pulse' pattern was anticipated to have comparable therapeutic effects to standard 130 Hz DBS, as it causes only a 0.77% reduction in the total number of pulses delivered over time. In contrast, the 'burst' pattern was anticipated to have minimal therapeutic effects relative to continuous DBS, as only 7.7% of the pulses are delivered, making it a useful probe for investigating activity in the absence of therapy. The evoked responses tend to increase in amplitude and sharpen across the consecutive pulses of a burst, and reach a steady state for longer duration stimuli.

We applied the burst stimulus to the STN of 12 PD patients (*n* = 23 hemispheres) undergoing DBS implantation surgery and observed ERNA of similar morphology in all cases, indicating it is a robust and reliable signal that can be measured across the patient population. As a control to ensure that ERNA was not a specious artefact, we also applied the burst stimulus to 3 Essential Tremor patients (*n* = 6 hemispheres) with electrodes implanted in the posterior subthalamic area (PSA), a white matter region medial to the STN. ERNA was not observed in the PSA, suggesting it is an electrophysiological response localizable to the STN.

### ERNA is localizable to the STN

To establish that ERNA varies with electrode position relative to the STN, we consecutively applied 10 s of burst stimulation to each of the four DBS electrodes whilst recording from the three unstimulated electrodes. The DBS implantation surgery aimed to position two of the four electrodes within the STN, with one electrode in the dorsal STN where DBS usually has greatest benefit and another in the ventral STN. This variance in electrode location facilitated comparison of ERNA responses from different regions of the STN with those outside of the nuclei. In 8 STN-PD patients (n = 16 hemispheres), we found that both ERNA amplitude and morphology varied depending on the stimulating and recording electrode positions. Figure 14 shows exemplar ERNA from the last pulse of each burst in one hemisphere, with the responses with the largest amplitude and most apparent decaying oscillation morphology occurring at the two middle electrodes in the target STN position. As a control, we also applied stimulation to two Essential Tremor patients (n = 4 hemispheres) using an implantation trajectory intended to position distal electrodes within the PSA and proximal electrodes in the ventral intermediate nucleus of the thalamus, another target for tremor that has previously been shown not to elicit evoked activity beyond ~2 ms.

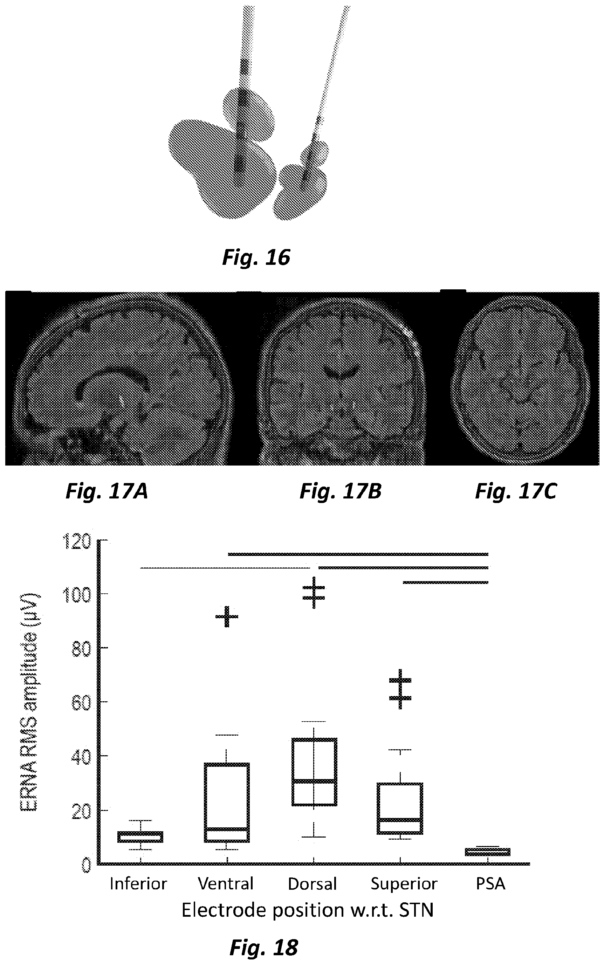

As variation in ERNA amplitude was the most apparent feature, we used it for further analysis. As not all recordings were in STN and contained distinct resonant activity, to quantify ERNA amplitude we calculated the root mean square (RMS) voltage over 4-20 ms. To estimate implanted electrode positions relative to the STN, 3D reconstructions (Fig. 16) were generated using post-operative CT scans merged with pre-operative MRI (Fig. 17). Electrodes were classified as being either superior to, inferior to, or within the STN, based on blinded measurements relative to the red nucleus. Within-STN electrodes were then further classified to be either dorsal or ventral.

ERNA amplitude varied significantly with electrode position (Kruskal-Wallis, H(4) = 45.73, p < 0.001), with only the inferior electrodes not significantly different from the PSA region (p = 0.370) (Fig. 18). Although dorsal electrodes tended to be higher than ventral and superior, they were not significantly different in amplitude from each other. To account for disparity in amplitude across patients due to variation in electrode positioning in the medial-lateral and posterior-anterior planes and underlying differences in patient physiology, we re-analyzed recordings from the STN-DBS electrodes after normalizing responses to the total ERNA amplitude across each hemisphere. After normalization, post hoc comparisons revealed a significant difference in amplitude between dorsal and ventral STN (Kruskal-Wallis, H(3) = 14.94, p = 0.002; Dunn's method post hoc, dorsal vs ventral: p = 0.043, dorsal vs superior: p = 0.081, dorsal vs inferior: p = 0.002).

These results show that ERNA is localizable to, and varies across, the STN, establishing its utility as a feedback signal for guiding electrode implantation to the most beneficial sites for stimulation. Furthermore, whilst variation in amplitude was the most apparent feature, other ERNA properties, such as frequency, latency, and rate of change, also have potential utility in discriminating STN regions.

### ERNA is modulated by DBS

To investigate whether ERNA was modulated by therapeutically effective DBS, we applied skipped-pulse stimuli of progressively increasing current amplitude (range 0.67-3.38 mA) in blocks of 60-90 s to 10 STN-PD patients (n = 19 hemispheres). In general, it was found that the second and subsequent peaks in ERNA were consistently observed to asymptotically increase in latency and spread further apart over time and as stimulation amplitudes increased, consistent with a decrease in the frequency of the resonant activity (Figures 5, 6, 21a to 21e). In many cases, the latency of the first peak also increased, although this change was not consistent across all recordings. The amplitude of the peaks was also generally observed to vary, often being greater at the beginning of each stimulation block and then gradually diminishing.

To quantify these effects, we calculated the inverse of the difference in latency between the first and second peaks as a representative measure of ERNA frequency. We also calculated the amplitude difference between the first peak and the first trough as a representative measure of ERNA amplitude. We then used averages of the 45-60 s period of each condition as estimates of asymptotic ERNA values for analysis.

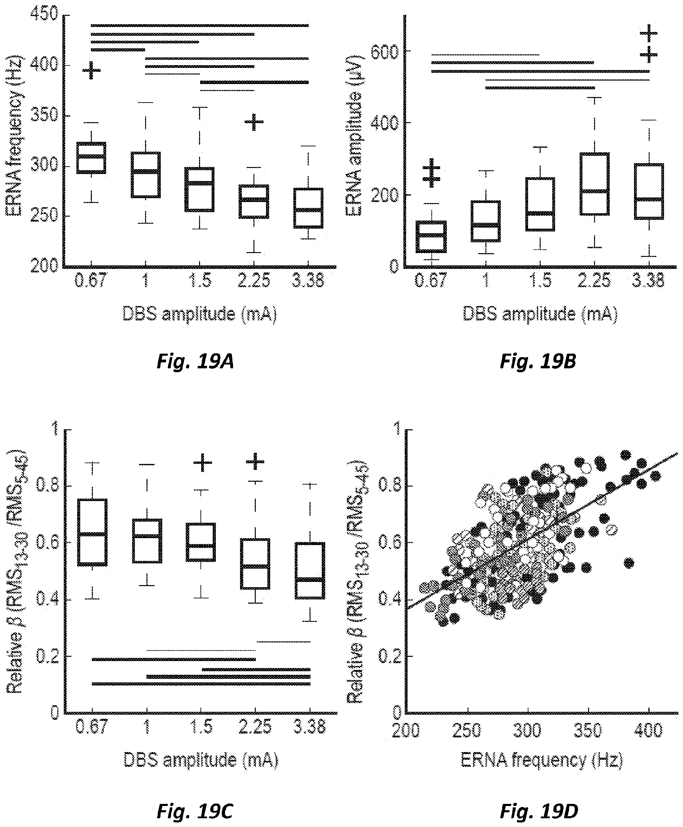

ERNA frequency significantly decreased across conditions (1-way Repeated Measures (RM) ANOVA, F(4,94) = 45.79, p < 0.001). Post hoc comparisons (Holm-Sidak) showed that ERNA frequency significantly decreased with each increasing step of DBS amplitude (Fig. 19A), except from 2.25 mA to 3.38 mA (p = 0.074). The median frequency was 256 Hz at 3.38 mA, approximately two times the stimulation rate of 130 Hz. It has been proposed that STN-DBS could act by creating a pacing effect within the globus pallidus interna at two times the stimulation rate, due to the immediate excitation of STN axons and inhibition/recovery time course of STN soma.

ERNA amplitude was also significantly different across conditions (Friedman, x²(4) = 41.31, p < 0.001). Tukey test post hoc comparisons indicated that ERNA amplitude initially increased with DBS amplitude and then plateaued at levels above 1.5 mA (Fig. 19B). While these effects may be related to the therapeutic effects of DBS, they may alternatively or additionally be due to saturation of neural firing in the STN. Due to this, we subsequently focused on ERNA frequency for correlation with therapeutic effects.

### ERNA correlates with therapeutic effects

The clinical efficacy of stimulation was confirmed by rating limb bradykinesia and rigidity according to the Unified Parkinson's Disease Rating Scale (UPDRS; items 22 and 23) immediately prior to stimulation and after 60 s at 2.25 mA. Both clinical signs improved significantly at 2.25 mA indicating that DBS was therapeutic (Wilcoxon Signed Rank, bradykinesia: Z = -3.62, p < 0.001, rigidity: Z = -3.70, p < 0.001).

However, time constraints precluded clinical examinations with each step in stimulation intensity. Therefore, to correlate ERNA modulation with patient state, we used beta-band (13-30 Hz) spontaneous LFP activity. Excessive synchronization of oscillations within the beta band has been strongly implicated in the pathophysiology of PD and its suppression has been correlated with improvement in movement impairments of bradykinesia and rigidity.

Using short-time Fourier transforms we calculated `relative beta', the RMS amplitude within the 13-30 Hz band divided by that within 5-45 Hz, as a representative measure of beta activity. We then averaged across the 45-60 s period of each stimulation amplitude condition and found relative beta to significantly vary (1-way RM ANOVA, F(4,89) = 18.11, p < 0.001). Post hoc tests (Holm-Sidak) showed significant suppression at 3.38 mA compared to all other conditions and at 2.25 mA compared to 0.67 and 1 mA (Fig. 19C). These results are consistent with previous studies showing that beta activity suppression by DBS correlates with improvement in clinical signs, and confirm that stimulation was therapeutically effective at and above 2.25 mA.

To further correlate ERNA frequency with beta activity, and thus therapeutic efficacy, we compared average values for 15 s non-overlapping blocks across each condition (Fig. 19B). ERNA frequency was significantly correlated with relative beta (Pearson product moment, *ρ* = 0.601, n = 90, p < 0.001).

These results indicate that ERNA is a clinically relevant biomarker. Furthermore, its large amplitude, ranging from 20 µVp-p to 681 µVp-p (median 146 µVp-p), is orders of magnitude larger than spontaneous beta LFP activity, whose absolute values ranged from 0.9 to 12.5 µVRMS (median 2.2 µVRMS). The robustness of ERNA and its distinct and gradually modulated morphology (Figs. 5, 6, 7, 21) contrast with the inherent variability in beta-band activity across patients and noisy bursting on-off nature of the beta-band signal. ERNA is therefore a more tractable signal to use with a fully-implantable DBS device compared to noisy, low-amplitude LFP measures.

### ERNA modulation washes out following DBS

Immediately before and after the therapeutic skipped-pulse stimulation we also applied 60 s of burst stimulation (hereafter referred to as pre- and post-DBS conditions), in order to monitor changes in activity as therapeutic effects washed out.

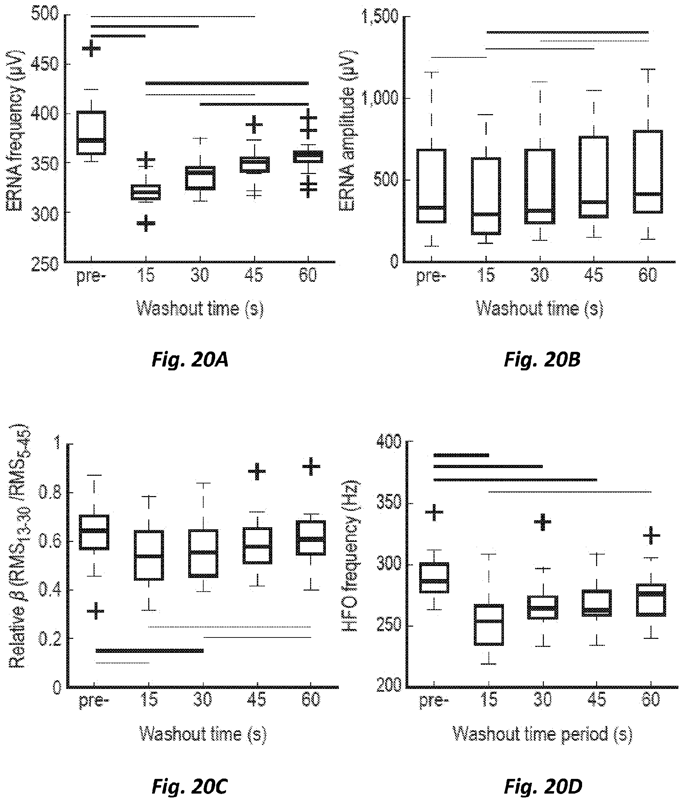

Generally, ERNA remained relatively stable pre-DBS, indicating the modulatory effects of burst stimulation were minimal. However, immediately post-DBS, ERNA peaks occurred at longer latencies before gradually returning towards their pre-DBS state. To quantify these effects, we averaged post-DBS ERNA frequency and amplitude over 15 s non-overlapping blocks and compared them to the last 15 s pre-DBS. Over all hemispheres tested (n = 19), differences were found in ERNA frequency (Friedman, χ²(4) = 70.23, p < 0.001), with frequencies at all time points significantly reduced compared to the pre-DBS frequency except for the final 45-60 s block (Tukey, p = 0.73) (Fig. 20A). ERNA amplitude also significantly varied across the time points (Friedman, χ²(4) = 31.37, p < 0.001), with differences between amplitudes at post-DBS time points indicating a washout of amplitude suppression caused by therapeutic stimulation (Fig. 20B). As the burst stimulus applied was constant across the pre- and post-DBS conditions, the variation observed in ERNA frequency and amplitude can be directly attributed to changes in the state of STN neural circuits as DBS effects washed out. Therapeutically effective DBS therefore modulates both ERNA frequency and amplitude, indicating ERNA has multiple properties that can feasibly be used as biomarkers and as tools for probing mechanisms of action.

We then assessed relative beta activity pre- and post-DBS and found it to be significantly different across time points (Friedman, χ²(4) = 24.55, p < 0.001). Consistent with previous reports, relative beta was significantly decreased immediately post-DBS and washed out to pre-DBS levels after 30 s (Fig. 20C). Supporting the skipped-pulse results, ERNA frequency was significantly correlated with relative beta pre- and post-DBS (Pearson product moment, *ρ* = 0.407, n = 152, p < 0.001). ERNA amplitude was also correlated with relative beta (Pearson product moment, *ρ* = 0.373, n = 152, p < 0.001), suggesting it too may have clinical and mechanistic relevance.

We also analyzed spontaneous LFP activity in the high frequency oscillation (HFO) band (200-400 Hz), which overlaps with the observed ERNA frequencies. Changes in the HFO band have been correlated with motor state and effective pharmacological therapy, particularly in conjunction with beta activity, and have been implicated in the mechanisms of action of DBS. Concurrent ERNA and HFO analysis was enabled by the use of burst stimulation, as data could be segmented to only include activity between bursts, thereby providing LFP epochs that were free of stimulation artefacts that can otherwise corrupt the HFO band.

As the HFO activity was generally characterized by a broadband peak in frequency, we calculated multitaper spectral estimates and then determined the frequency and amplitude of the peak occurring between 200-400 Hz. Comparing averages across 15 s non-overlapping blocks (Fig. 20D), we found HFO peak frequency to be significantly decreased post-DBS (Friedman, χ²(4) = 45.18, p < 0.001), until the final 45-60 s block (Tukey, p = 0.077). This washout trend matches that for ERNA frequency, albeit at a lower frequency, with a significant correlation between the two (Pearson product moment, *ρ* = 0.546, n = 152, p < 0.001). The median HFO peak frequency immediately post-DBS was 253 Hz, comparable to the median ERNA frequency of the therapeutic 3.38 mA condition (256 Hz), suggesting HFO activity occurs at the same frequency as ERNA during the more continuous skipped-pulse stimulation.

No significant differences were found in HFO peak amplitude (Friedman, χ²(4) = 2.11, p = 0.72), although it did significantly correlate with ERNA amplitude (Pearson product moment, *ρ* = 0.429, n = 152). It is likely that the very small amplitude (<1 µV) of HFO peaks resulted in any modulatory effects being obscured by noise in the recordings.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

## Claims

1. A neurostimulation system (90), comprising:
at least one electrode (72) adapted for implantation in or near a target neural structure in the brain;
a signal generator (94) selectively coupled to one or more of the at least one electrode and configured to apply a plurality of electrical stimuli having differing amplitudes via a first electrode of the at least one electrode at a first position in a brain of a patient;
a measurement device (96) selectively coupled to one or more of the at least one electrode and configured to detect, for each of the plurality of electrical stimuli, a resonant response from the target neural structure, each resonant response evoked by a respective one of the plurality of electrical stimuli; and
a processing unit (92) coupled to the measurement device and configured to:
determine a change in one or more common waveform characteristics between two or more of the detected resonant responses; and
determine an effectiveness of delivering therapeutic stimulation at the first position based on the change.

2. The system of claim 1, wherein the one or more common waveform characteristics comprises one or more of the following:
a) an amplitude of the resonant response;
b) a resurgence of the resonant response;
c) a frequency of the resonant response;
d) a temporal envelope of the resonant response;
e) a fine structure of the resonant response;
f) a rate of decay of the resonant response;
g) a delay between the onset of the stimulus and the onset of a temporal feature of the resonant response amplitude.

3. The system of claims 1 or 2, wherein the processing unit is further configured to:
select an amplitude of the differing amplitudes for therapeutic stimulation of the target neural structure based on the change.

4. The system of any one of claims 1 to 3, wherein the processing unit is further configured to:
select the first position for therapeutic stimulation of the target neural structure based on the change.

5. The system of any one of claims 1 to 4, wherein:
the at least one electrode comprises a first electrode at the first position and a second electrode at a second position different from the first position;
the signal generator is configured to apply the plurality of electrical stimuli to each of the first and second electrodes;
the measurement device is configured to detect the resonant response from the target neural structure evoked by the plurality of electrical stimuli applied to each of the first and second electrodes; and
the processing unit is configured to:
determine a change in one or more common waveform characteristics between two or more of the detected resonant responses evoked by each of the plurality of electrical stimuli applied to the second electrode in the second position;
compare the change in the one or more common waveform characteristics of the two or more of the detected resonant responses at the first electrode to a change in one or more common waveform characteristics between two or more of the detected resonant responses at the second electrode.

6. The system of any one of claims 4 or 5, wherein the processing unit is configured to select one of the first electrode and the second electrode for therapeutic stimulation of target neural structure based on the comparison.

7. The system of claim 1, wherein in response to moving of the first electrode to a second position in the brain different from the first position, the signal generator is configured to apply the plurality of electrical stimuli to the first electrode in the second position;
wherein the measurement device is configured to detect the resonant response from the target neural structure evoked by each of the plurality of electrical stimuli applied to the first electrode in the second position; and
wherein the processing unit is configured to:
determine a change in one or more common waveform characteristics between two or more of the detected resonant responses evoked by each of the plurality of electrical stimuli applied to the first electrode in the second position;
compare the change in the one or more common waveform characteristics between the two or more of the detected resonant responses detected at the first position to a change in the one or more common waveform characteristics between the two or more of the detected resonant responses detected at second position; and
determine an optimum electrode position for therapeutic stimulation of target neural structure based on the comparison.

8. The system of any one of claims 1 to 7, wherein determining the change in the one or more common waveform characteristics between the two or more of the detected resonant responses comprises determining a rate of change in the one or more of the common waveform characteristics between the two or more of the detected resonant responses.

9. The system of any one of claims 1 to 4, wherein the plurality of electrical stimuli are applied at the first location by two or more electrodes of the at least one electrode.

10. The system of claim 9, wherein at least one of the two or more electrodes is not located at the first position.

11. The system of any one of claims 1 to 4, wherein the resonant response is detected at two or more of the at least one electrode.

12. The system of any one of the preceding claims, wherein the patient is under general anaesthetic.

13. A non-therapeutic method, comprising:
a. applying a plurality of electrical stimuli having differing amplitudes via a first electrode at a first position in a brain of a patient, a total energy provided to the patient by the plurality of electrical stimuli being below a therapeutic threshold;
b. for each of the plurality of electrical stimuli, detecting a resonant response from a target neural structure in the brain, each resonant response evoked by a respective one of the electrical stimuli;
c. determining a change in one or more common waveform characteristics between two or more of the detected resonant responses; and
d. determining an effectiveness of delivering therapeutic stimulation at the first position based on the change.

## Patentansprüche

1. Neurostimulationssystem (90), umfassend:
mindestens eine Elektrode (72), die zur Implantation in oder in der Nähe einer Nervenzielstruktur in dem Gehirn angepasst ist;
einen Signalgenerator (94), der selektiv mit einer oder mehreren der mindestens einen Elektrode gekoppelt und konfiguriert ist, um eine Vielzahl von elektrischen Reizen mit unterschiedlichen Amplituden mittels einer ersten Elektrode der mindestens einen Elektrode an einer ersten Position in einem Gehirn eines Patienten anzuwenden;
eine Messvorrichtung (96), die selektiv mit einer oder mehreren der mindestens einen Elektrode gekoppelt und konfiguriert ist, um für jeden der Vielzahl von elektrischen Reizen eine Resonanzantwort von der Nervenzielstruktur zu detektieren, wobei jede Resonanzantwort durch einen jeweiligen einen der Vielzahl von elektrischen Reizen hervorgerufen wird; und
eine Verarbeitungseinheit (92), die mit der Messvorrichtung gekoppelt und konfiguriert ist zum:
Bestimmen einer Veränderung in einer oder mehreren gemeinsamen Wellenformeigenschaften zwischen zwei oder mehr der detektierten Resonanzantworten; und
Bestimmen einer Wirksamkeit des Abgebens von therapeutischer Stimulation an der ersten Position basierend auf der Veränderung.

2. System nach Anspruch 1, wobei die eine oder mehreren gemeinsamen Wellenformeigenschaften eines oder mehrere des Folgenden umfassen:
a) eine Amplitude der Resonanzantwort;
b) einen Wiederanstieg der Resonanzantwort;
c) eine Frequenz der Resonanzantwort;
d) eine zeitliche Hüllkurve der Resonanzantwort;
e) eine Feinstruktur der Resonanzantwort;
f) eine Abklingrate der Resonanzantwort;
g) eine Verzögerung zwischen dem Beginn des Reizes und dem Beginn eines zeitlichen Merkmals der Resonanzantwortamplitude.

3. System nach Ansprüchen 1 oder 2, wobei die Verarbeitungseinheit ferner konfiguriert ist zum:
Auswählen einer Amplitude der sich unterscheidenden Amplituden zur therapeutischen Stimulation der Nervenzielstruktur basierend auf der Veränderung.

4. System nach einem der Ansprüche 1 bis 3, wobei die Verarbeitungseinheit ferner konfiguriert ist zum:
Auswählen der ersten Position zur therapeutischen Stimulation der Nervenzielstruktur basierend auf der Veränderung.

5. System nach einem der Ansprüche 1 bis 4, wobei:
die mindestens eine Elektrode eine erste Elektrode an der ersten Position und eine zweite Elektrode an einer zweiten Position umfasst, die sich von der ersten Position unterscheidet;
der Signalgenerator konfiguriert ist, um die Vielzahl von elektrischen Reizen an jeder der ersten und zweiten Elektrode anzuwenden;
die Messvorrichtung konfiguriert ist, um die Resonanzantwort von der Nervenzielstruktur zu detektieren, die durch die Vielzahl von elektrischen Reizen hervorgerufen wird, die an jeder der ersten und zweiten Elektrode angewendet werden; und
die Verarbeitungseinheit konfiguriert ist zum:
Bestimmen einer Veränderung in einer oder mehreren gemeinsamen Wellenformeigenschaften zwischen zwei oder mehr der detektierten Resonanzantworten, die durch jeden der Vielzahl von elektrischen Reizen hervorgerufen werden, die an der zweiten Elektrode in der zweiten Position angewendet werden;
Vergleichen der Veränderung der einen oder mehreren gemeinsamen Wellenformeigenschaften der zwei oder mehr der an der ersten Elektrode detektierten Resonanzantworten mit einer Veränderung in einer oder mehreren gemeinsamen Wellenformeigenschaften zwischen zwei oder mehr der an der zweiten Elektrode detektierten Resonanzantworten.

6. System nach einem der Ansprüche 4 oder 5, wobei die Verarbeitungseinheit konfiguriert ist, um basierend auf dem Vergleich eine der ersten Elektrode und der zweiten Elektrode zur therapeutischen Stimulation der Nervenzielstruktur auszuwählen.

7. System nach Anspruch 1, wobei der Signalgenerator als Reaktion auf das Bewegen der ersten Elektrode zu einer zweiten Position in dem Gehirn, die sich von der ersten Position unterscheidet, konfiguriert ist, um die Vielzahl von elektrischen Reizen an der ersten Elektrode in der zweiten Position anzuwenden;
wobei die Messvorrichtung konfiguriert ist, um die Resonanzantwort von der Nervenzielstruktur zu detektieren, die durch jeden der Vielzahl von elektrischen Reizen hervorgerufen wird, die an der ersten Elektrode in der zweiten Position angewendet werden; und
wobei die Verarbeitungseinheit konfiguriert ist zum:
Bestimmen einer Veränderung in einer oder mehreren gemeinsamen Wellenformeigenschaften zwischen zwei oder mehr der detektierten Resonanzantworten, die durch jeden der Vielzahl von elektrischen Reizen hervorgerufen werden, die an der ersten Elektrode in der zweiten Position angewendet werden;
Vergleichen der Veränderung in der einen oder den mehreren gemeinsamen Wellenformeigenschaften zwischen den zwei oder mehr der detektierten Resonanzantworten, die an der ersten Position detektiert wurden, mit einer Veränderung in der einen oder den mehreren gemeinsamen Wellenformeigenschaften zwischen den zwei oder mehr der detektieren Resonanzantworten, die an zweiter Position detektiert wurden; und
Bestimmen einer optimalen Elektrodenposition zur therapeutischen Stimulation der Nervenzielstruktur basierend auf dem Vergleich.

8. System nach einem der Ansprüche 1 bis 7, wobei das Bestimmen der Veränderung in der einen oder den mehreren gemeinsamen Wellenformeigenschaften zwischen den zwei oder mehr der detektierten Resonanzantworten das Bestimmen einer Rate der Veränderung in der einen oder den mehreren der gemeinsamen Wellenformeigenschaften zwischen den zwei oder mehr der detektierten Resonanzantworten umfasst.

9. System nach einem der Ansprüche 1 bis 4, wobei die Vielzahl von elektrischen Reizen an der ersten Stelle durch zwei oder mehr Elektroden der mindestens einen Elektrode angewendet werden.

10. System nach Anspruch 9, wobei sich mindestens eine der zwei oder mehr Elektroden nicht an der ersten Position befindet.

11. System nach einem der Ansprüche 1 bis 4, wobei die Resonanzantwort an zwei oder mehr der mindestens einen Elektrode detektiert wird.

12. System nach einem der vorstehenden Ansprüche, wobei der Patient unter Vollnarkose ist.

13. Nicht-therapeutisches Verfahren, umfassend:
a. Anwenden einer Vielzahl von elektrischen Reizen mit unterschiedlichen Amplituden mittels einer ersten Elektrode an einer ersten Position in einem Gehirn eines Patienten, wobei eine dem Patienten durch die Vielzahl von elektrischen Reizen bereitgestellte Gesamtenergie unterhalb einer therapeutischen Schwelle liegt;
b. für jeden der Vielzahl von elektrischen Reizen, Detektieren einer Resonanzantwort von einer Nervenzielstruktur in dem Gehirn, wobei jede Resonanzantwort durch einen jeweiligen einen der elektrischen Reize hervorgerufen wird;
c. Bestimmen einer Veränderung in einer oder mehreren Wellenformeigenschaften zwischen zwei oder mehr der detektierten Resonanzantworten; und
d. Bestimmen einer Wirksamkeit des Abgebens von therapeutischer Stimulation an der ersten Position basierend auf der Veränderung.

## Revendications

1. Système de neurostimulation (90), comprenant :
au moins une électrode (72) adaptée pour une implantation dans ou à proximité d'une structure neurale cible dans le cerveau ;
un générateur de signal (94) couplé sélectivement à une ou plusieurs de l'au moins une électrode et configuré pour appliquer une pluralité de stimuli électriques ayant des amplitudes différentes via une première électrode de l'au moins une électrode à une première position dans un cerveau d'un patient ;
un dispositif de mesure (96) couplé sélectivement à une ou plusieurs de l'au moins une électrode et configuré pour détecter, pour chacun de la pluralité de stimuli électriques, une réponse résonante à partir de la structure neurale cible, chaque réponse résonante étant évoquée par un stimulus respectif de la pluralité de stimuli électriques ; et
une unité de traitement (92) couplée au dispositif de mesure et configurée pour :
déterminer un changement d'une ou de plusieurs caractéristiques de forme d'onde communes entre deux ou plusieurs des réponses résonantes détectées ; et
déterminer une efficacité d'administration de stimulation thérapeutique au niveau de la première position sur la base du changement.

2. Système selon la revendication 1, dans lequel les une ou plusieurs caractéristiques de forme d'onde communes comprennent un ou plusieurs parmi :
a) une amplitude de la réponse résonante ;
b) une résurgence de la réponse résonante ;
c) une fréquence de la réponse résonante ;
d) une enveloppe temporelle de la réponse résonante ;
e) une structure fine de la réponse résonante ;
f) un taux de décroissance de la réponse résonante ;
g) un délai entre le début du stimulus et le début d'une caractéristique temporelle de l'amplitude de la réponse résonante.

3. Système selon la revendication 1 ou 2, dans lequel l'unité de traitement est en outre configurée pour :
sélectionner une amplitude parmi les différentes amplitudes pour une stimulation thérapeutique de la structure neuronale cible sur la base du changement.

4. Système selon l'une quelconque des revendications 1 à 3, dans lequel l'unité de traitement est en outre configurée pour :
sélectionner la première position pour une stimulation thérapeutique de la structure neuronale cible sur la base du changement.

5. Système selon l'une quelconque des revendications 1 à 4, dans lequel :
l'au moins une électrode comprend une première électrode au niveau de la première position et une seconde électrode au niveau d'une seconde position différente de la première position ;
le générateur de signal est configuré pour appliquer la pluralité de stimuli électriques à chacune des première et seconde électrodes ;
le dispositif de mesure est configuré pour détecter la réponse résonante provenant de la structure neuronale cible évoquée par la pluralité de stimuli électriques appliqués à chacune des première et seconde électrodes ; et
l'unité de traitement est configurée pour :
déterminer un changement dans une ou plusieurs caractéristiques de forme d'onde communes entre deux ou plusieurs des réponses résonantes détectées évoquées par chacun de la pluralité de stimuli électriques appliqués à la seconde électrode dans la seconde position ;
comparer le changement des une ou plusieurs caractéristiques de forme d'onde communes des deux ou plusieurs des réponses résonantes détectées au niveau de la première électrode à un changement d'une ou plusieurs caractéristiques de forme d'onde communes entre deux ou plusieurs des réponses résonantes détectées au niveau de la seconde électrode.

6. Système selon l'une quelconque des revendications 4 ou 5, dans lequel l'unité de traitement est configurée pour sélectionner l'une parmi la première électrode et la seconde électrode pour une stimulation thérapeutique de la structure neuronale cible sur la base de la comparaison.

7. Système selon la revendication 1, dans lequel en réponse au déplacement de la première électrode vers une seconde position dans le cerveau différente de la première position, le générateur de signal est configuré pour appliquer la pluralité de stimuli électriques à la première électrode dans la seconde position ;
dans lequel le dispositif de mesure est configuré pour détecter la réponse résonante provenant de la structure neuronale cible évoquée par chacun de la pluralité de stimuli électriques appliqués à la première électrode dans la seconde position ; et
dans lequel l'unité de traitement est configurée pour :
déterminer un changement dans une ou plusieurs caractéristiques de forme d'onde communes entre deux ou plusieurs des réponses résonantes détectées évoquées par chacun de la pluralité de stimuli électriques appliqués à la première électrode dans la seconde position ;
comparer le changement des une ou plusieurs caractéristiques de forme d'onde communes entre les deux ou plusieurs des réponses résonantes détectées au niveau de la première position à un changement des une ou plusieurs caractéristiques de forme d'onde communes entre les deux ou plusieurs des réponses résonantes détectées au niveau de la seconde position ; et
déterminer une position d'électrode optimale pour une stimulation thérapeutique de la structure neuronale cible sur la base de la comparaison.

8. Système selon l'une quelconque des revendications 1 à 7, dans lequel la détermination du changement des une ou plusieurs caractéristiques de forme d'onde communes entre les deux ou plusieurs des réponses résonantes détectées comprend la détermination d'un taux de changement des une ou plusieurs caractéristiques de forme d'onde communes entre les deux ou plusieurs des réponses résonantes détectées.

9. Système selon l'une quelconque des revendications 1 à 4, dans lequel la pluralité de stimuli électriques sont appliqués au premier emplacement par deux ou plusieurs électrodes de l'au moins une électrode.

10. Système selon la revendication 9, dans lequel au moins une des deux ou plusieurs électrodes n'est pas située dans la première position.

11. Système selon l'une quelconque des revendications 1 à 4, dans lequel la réponse résonante est détectée au niveau de deux ou plusieurs de l'au moins une électrode.

12. Système selon l'une quelconque des revendications précédentes, dans lequel le patient est sous anesthésie générale.

13. Procédé non thérapeutique, comprenant :
a. l'application d'une pluralité de stimuli électriques ayant des amplitudes différentes via une première électrode à une première position dans un cerveau d'un patient, une énergie totale fournie au patient par la pluralité de stimuli électriques étant inférieure à un seuil thérapeutique ;
b. pour chacun de la pluralité de stimuli électriques, la détection d'une réponse résonante provenant d'une structure neuronale cible dans le cerveau, chaque réponse résonnante étant évoquée par un stimulus respectif des stimuli électriques ;
c. la détermination d'un changement d'une ou de plusieurs caractéristiques de forme d'onde communes entre deux ou plusieurs des réponses résonantes détectées ; et
d. la détermination d'une efficacité d'administration d'une stimulation thérapeutique au niveau de la première position sur la base du changement.
que.
